# EUROPEAN PATENT SPECIFICATION

(11) **EP 1 811 013 B1**
(45) Date of publication and mention of the grant of the patent: **12.08.2009**
(21) Application number: 06256249.1
(22) Date of filing: 20.12.2006
(51) Int. Cl.: C11D 1/00, C11D 1/02, C11D 1/38, C11D 1/65, C11D 3/00, C11D 3/20

(54) **Foamable alcoholic composition**
Verschäumbare Alkoholzusammensetzung
Composition alcoolique moussante

(30) Priority: 28.12.2005 US 754536 P; 22.05.2006 US 438664
(43) Date of publication of application: 25.07.2007
(73) Proprietor: Gojo Industries, Inc., Akron, OH 44311 (US)
(72) Inventor: Snyder, Marcia, Stow Ohio 44224 (US); Viscovitz, John H., Akron Ohio 44319 (US)
(74) Representative: Makovski, Priscilla Mary

(56) References cited:
- EP-A2- 0 689 767
- WO-A-20/05030917
- WO-A-20/06066888
- WO-A-20/06094387
- US-A- 5 167 950

## Description

This application gains priority from U.S. Provisional Application No. 60/754,536, filed December 28, 2005.

### TECHNICAL FIELD

This invention relates to foamable alcoholic compositions, and more particularly, to foamable alcoholic compositions that include a foaming surfactant selected from fluorosurfactants, siloxane polymer surfactants, and combinations thereof.

### BACKGROUND OF THE INVENTION

Foam cleaning products are popular, in part because they are easier to spread on surfaces. Consumers seem to prefer the luxury of foamed soap products. Less foam is needed to produce the same cleaning power as liquids or gels, due at least partly to the higher surface area of the foam. Properly formulated foam products do not produce the drip and splash that is experienced with traditional gelled or liquid products, due to the higher surface tension of the foam. This prevents damage to the floors and walls of facilities where the product dispensers are used. Manufacturing of foam products may be easier than gelled products, which often incorporate powdered thickeners that are difficult to handle.

Aqueous foam cleaning products have been described, for example, a foam cleaning composition containing a surfactant, a foam-boosting solvent such as a glycol, and at least 80 percent by weight (wt. %) water.

Alcoholic products are popular as sanitizers for the skin. However, foaming products based upon alcoholic compositions are problematic, because alcohol is known to have strong defoaming properties. Although aerosol-based alcoholic foams are available, these aerosol products are generally more expensive to manufacture than non-aerosol foams.

A stable foamed composition containing water and an alkoxylated silicone compound has been described that may contain up to 30 wt. % alcohol, and may be stable for one month or longer. However, alcoholic compositions do not exhibit effective antimicrobial properties unless the alcohol is present in an amount of 50 weight percent or more.

A foamable liquid cleanser containing a surfactant, a humectant, water, and from about 35 to about 70 wt. % alcohol has been described, however the foam produced when this composition is aerated collapses substantially to liquid within about 5 to 45 seconds.

A skin disinfecting formulation has been described that comprises: (a) an alcohol in an amount from about 50 to about 80 weight percent of the total composition; (b) from about 0.02 wt. % to about 5 wt. % of a block copolymer; (c) from about 5 wt. % to about 25 wt. % of a foaming surfactant; (d) from about 0 wt. % to about 3 wt. % of a thickening agent; (e) from about 1 wt. % to about 5 wt. % of an emulsifier; (f) from about 0wt. % to about 5wt. % of a preservative; (g) from about 1 wt. % to about 10 wt. % of a cleaning agent; (h) from about 0.5 wt. % to about 5 wt. % of a polyalkylene glycol; (i) from about 0.05 wt. % to about 5 wt. % of a moisturizer and/or emollient; and (j) from about 6 wt. % to about 30 wt. % of water. The foaming surfactants that are taught are ammonium fatty sulfo succinate, cocamide dea, alkonolamides such as cocodiethanolamide, amine oxides such as cetyldimethyl amino oxide and amphoterics such as isostearoamphoropionate and lauramidopropyl betaine surfactant.

International Patent Application Publication No. W02006/094387 relates to compositions containing high contents of lower alcohol (C₁₋₄) and a silicone-based surfactant that can be dispensed as a foam under low pressure conditions from unpressurized containers.

International Patent Application Publication No. W02006/066888 teaches a foam composition containing; a.) at least 52-99 weight percent of an alcohol, b.) a surfactant or a surfactant mixture, and c.) at least one polyalkylene glycol.

U.S. Patent No. 5,167,960 teaches a high-alcohol content aerosol antimicrobial mousse. The mousse composition comprises a hydrocarbon propellant and a concentrate that includes from 52-75 percent by weight ethanol or isopropyl alcohol, a water-dispersible polymeric gelling agent, and an amphiphilic system that includes an alcohol having from 16-22 carbons and a non-ionic surfactant

International Patent Application Publication No. W02005/030917 teaches compositions with high contents of lower alcohol (C₁₋₄) that can be a gel-like composition or a solution able to be dispensed as a foam. The compositions to be dispensed as foams contain a fluorosurfactant.

Commercially available non-aerosol alcoholic foams have been formulated with specific perfluoroalkyl phosphate surfactants and dispensed from a foaming pump mechanism. But the foam thus produced is fleeting, and will immediately begin to deflate or liquefy into a water-thin liquid. When the alcoholic foam is dispensed from a common foaming pump mechanism, some of the foam is left within the tubes of the dispenser, and, as the foam breaks down, the composition often drips out of the dispenser. This dripping not only wastes product, it makes the area around the dispenser look unsanitary and may result in damage to the walls and floors, as well as resulting in a slip hazard if the product is dripping on the floor. Thus, there is a need for stabilized foamable alcoholic compositions.

### Summary of the invention

One or more embodiments of this invention provide a foamable composition as set out in claim 1.

One or more embodiments of this invention provide a method for reducing dripping of a composition from a forming pump dispenser adapted to dispense said composition as set out in claim 2.

One or more embodiments of this invention provide a method for stabilizing a foam formed from a foamable composition as set out in claim 3.

One or more embodiments of this invention provide an antimicrobial composition as set out in claim 10.

One or more embodiments of this invention provide a foamable composition as set out in claim 11.

### BRIEF DESCRIPTION OF THE DRAWINGS

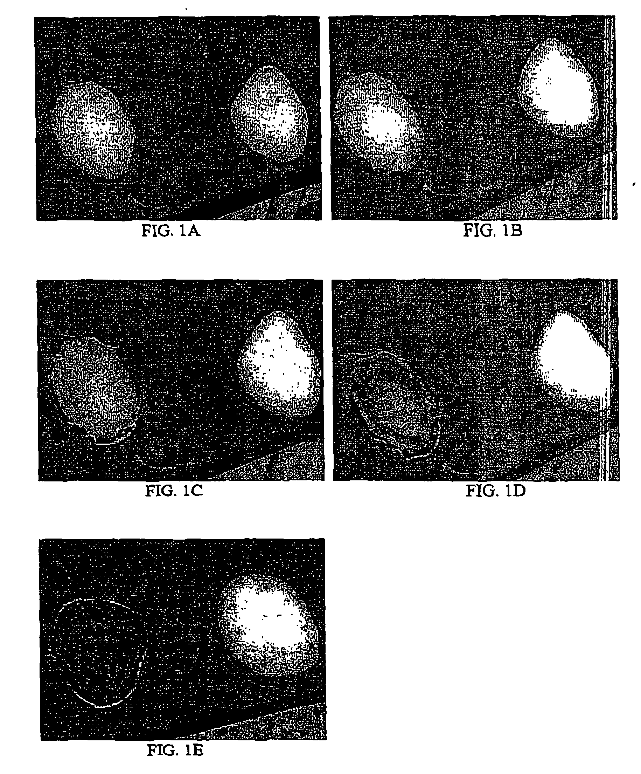

FIG. 1A is a photograph showing a surface with a stabilized foamed composition on the right and an unstabilized foamed composition on the left, immediately after the foams were dispensed.

FIG. 1B is a photograph showing a surface with a stabilized foamed composition on the right and an unstabilized foamed composition on the left, 1 minute after the foams were dispensed.

FIG. 1C is a photograph showing a surface with a stabilized foamed composition on the right and an unstabilized foamed composition on the left, 3 minutes after the foams were dispensed.

FIG. 1D is a photograph showing a surface with a stabilized foamed composition on the right and an unstabilized foamed composition on the left, 5 minutes after the foams were dispensed.

FIG. 1E is a photograph showing a surface with a stabilized foamed composition on the right and an unstabilized foamed composition on the left, 1 hour after the foams were dispensed.

### DETAILED DESCRIPTION OF ILLUSTRATIVE EMBODIMENTS

Foamable alcoholic compositions in accordance with this invention include at least one alcohol and at least one foaming surfactant. In one embodiment, the alcohol is a lower alkanol, i.e. an alcohol containing 1 to 4 carbon atoms. Typically, these alcohols have antimicrobial properties. Examples of lower alkanols include, but are not limited to, methanol, ethanol, propanol, isopropanol, butanol, isobutanol, tertiary butanol, and mixtures thereof. In one embodiment, the alcohol comprises ethanol.

Generally, the alcoholic composition comprises an amount of alcohol of at least about 40 weight percent (wt. %), based upon the total weight of the alcoholic composition. In one embodiment, the alcoholic composition comprises at least about 45 weight percent alcohol, in another embodiment, the alcoholic composition comprises at least about 50 weight percent alcohol, and in yet another embodiment, the alcoholic composition comprises at least about 60 weight percent alcohol, based upon the total weight of alcoholic composition. More or less alcohol may be required in certain instances, depending particularly on other ingredients and/or the amounts thereof employed in the composition. In certain embodiments, the alcoholic composition comprises from about 40 weight percent to about 98 weight percent alcohol, in other embodiments, the alcoholic composition comprises from about 45 weight percent to about 95 weight percent of alcohol, in yet other embodiments, the alcoholic composition comprises from about 50 weight percent to about 90 weight percent of alcohol, and in still other embodiments, the alcoholic composition comprises from about 60 weight percent to about 80 weight percent of alcohol, based upon the total weight of the alcoholic composition.

The foaming surfactant contributes foaming properties to the alcoholic composition, and may include anionic, cationic, nonionic, zwitterionic, or amphoteric surfactants and their associated salts. In one embodiment, the foaming surfactant includes a fluorosurfactant, a siloxane polymer surfactant, or a combination thereof. Fluorosurfactants include compounds that contain at least one fluorine atom. Examples of fluorosurfactants include perfluoroalkylethyl phosphates, perfluoroalkylethyl betaines, fluoroaliphatic amine oxides, fluoroaliphatic sodium sulfosuccinates, fluoroaliphatic stearate esters, fluoroaliphatic phosphate esters, fluoroaliphatic quaternaries, fluoroaliphatic polyoxyethylenes, and the like, and mixtures thereof.

In one embodiment, the fluorosurfactant contains a charged species, i.e. is anionic, cationic, or zwitterionic. Examples of fluorosurfactants containing a charged species include perfluoroalkylethyl phosphates, perfluoroalkylethyl betaines, fluoroaliphatic amine oxides, fluoroaliphatic sodium sulfosuccinates, fluoroaliphatic phosphate esters, and fluoroaliphatic quaternaries. Specific examples of fluorosurfactants include DEA-C8-18 perfluoroalkylethyl phosphate, TEA-C8-18 perfluoroalkylethyl phosphate, NH₄-C8-18 perfluoroalkylethyl phosphate, and C8-18 perfluoroalkylethyl betaine.

In one embodiment, the fluorosurfactant includes a compound that may be represented by the formula

[F₃CF₂C - (CF₂CF₂)ₓ -CH₂CH₂-O-P₂O₃]⁻ [R¹ ] ⁺

where [R¹] ⁺ includes DEA, TEA, NH₄, or betaine, and where x is an integer from about 4 to about 18.

Siloxane polymer surfactants may be generally characterized by containing one or more Si-O-Si linkages in the polymer backbone. The siloxane polymer surfactant may or may not include a fluorine atom. Therefore, some foaming surfactants may be classified as both fluorosurfactants and siloxane polymer surfactants. Siloxane polymer surfactants include organopolysiloxane dimethicone polyols, silicone carbinol fluids, silicone polyethers, alkylmethyl siloxanes, amodimethicones, trisiloxane ethoxylates, dimethiconols, quaternized silicone surfactants, polysilicones, silicone crosspolymers, and silicone waxes.

Examples of siloxane polymer surfactants include dimethicone PEG-7 undecylenate, PEG-10 dimethicone, PEG-8 dimethicone, PEG-12 dimethicone, perfluorononylethyl carboxydecal PEG 10, PEG-20/PPG-23 dimethicone, PEG-11 methyl ether dimethicone, bis-PEG/PPG-20/20 dimethicone, silicone quats, PEG-9 dimethicone, PPG-12 dimethicone, fluoro PEG-8 dimethicone, PEG 23/PPG 6 dimethicone, PEG 20/PPG 23 dimethicone, PEG 17 dimethicone, PEG5/PPG3 methicone, bis PEG20 dimethicone, PEG/PPG20/15 dimethicone copolyol and sulfosuccinate blends, PEG-8 dimethicone\dimmer acid blends, PEG-8 dimethicone\fatty acid blends, PEG-8 dimethicone\cold pressed vegetable oil\polyquaternium blends, random block polymers and mixtures thereof.

In one embodiment, the siloxane polymer surfactant includes a compound that may be represented by the formula

R² - Si (CH₃)₂ - [O-Si (CH₃)₂]*ₐ* - (O - Si (CH₃) R³]*_{b}* - O - Si (CH₃)₂ - R²

where R² and R³ independently include a methyl group or a moiety that may be represented by the formula

-(CH₂)₃ - O - (CH₂CH₂O)*_{c}* - [CH₂CH(CH₃)O]*_{d}* - (CH₂CH₂O)*ₑ* H

with the proviso that both R² and R³ are not CH₃ , where a is an integer from about 3 to about 21, b is an integer from about 1 to about 7, c is an integer from about 0 to about 40, d is an integer from about 0 to about 40, and e is an integer from about 0 to about 40, with the proviso that a ≥ 3 x b and that c + d + e ≥ 5.

The amount of foaming surfactant is not particularly limited, so long as an effective amount to produce foaming is present. In certain embodiments, the effective amount to produce foaming may vary, depending upon the amount of alcohol and other ingredients that are present. In one or more embodiments, the alcoholic composition includes at least about 0.002 wt. % of foaming surfactant, based upon the total weight of the alcoholic composition. In another embodiment, the alcoholic composition includes at least about 0.01 wt. % of foaming surfactant, based upon the total weight of the alcoholic composition. In yet another embodiment, the alcoholic composition includes at least about 0.05 wt. % of foaming surfactant, based upon the total weight of the alcoholic composition.

In one embodiment, the foaming surfactant is present in an amount of from about 0.002 to about 4 weight percent, based upon the total weight of the alcoholic composition. In another embodiment, the foaming surfactant is present in an amount of from about 0.01 to about 2 weight percent, based upon the total weight of the alcoholic composition. It is envisioned that higher amounts may also be effective to produce foam. All such weights as they pertain to listed ingredients are based on the active level and, therefore, do not include carriers or by-products that may be included in commercially available materials, unless otherwise specified.

In some embodiments, for economic or other reasons it may be desirable to limit the amount of fluorosurfactant used. Advantageously, stable foam can be produced from a composition according to the present invention containing greater than about 60 wt. % alcohol, and from about 0.002 to about 0.5 wt. % fluorosurfactant, based upon the total weight of the composition. In certain embodiments, the foamable composition includes greater than about 65 wt. % alcohol, and from about 0.002 to about 0.4 wt. % fluorosurfactant, based upon the total weight of the composition.

In other embodiments, it may be desirable to use higher amounts of foaming surfactant. For example, in certain embodiments where the foaming alcoholic composition of the present invention includes a cleansing or sanitizing product that is applied to a surface and then rinsed off, higher amounts of foaming surfactant may be employed. In these embodiments, the amount of foaming surfactant is present in amounts up to about 35 wt. %, based upon the total weight of the composition.

In one or more embodiments, the foaming surfactant is added directly to the alcoholic composition. In other embodiments, the foaming surfactant is added to the alcoholic composition as a solution or emulsion. In other words, the foaming surfactant may be premixed with a carrier to form a foaming surfactant solution or emulsion, with the proviso that the carrier does not deleteriously affect the foaming properties of the alcoholic composition. Examples of carriers include water, alcohol, glycols such as propylene or ethylene glycol, ketones, linear and/or cyclic hydrocarbons, triglycerides, carbonates, silicones, alkenes, esters such as acetates, benzoates, fatty esters, glyceryl esters, ethers, amides, polyethylene glycols and PEG/PPG copolymers, inorganic salt solutions such as saline, and mixtures thereof. It will be understood that, when the foaming surfactant is premixed to form a foaming surfactant solution or emulsion, the amount of solution or emulsion that is added to the alcoholic composition may be selected so that the amount of foaming surfactant falls within the ranges set forth hereinabove.

Foaming surfactant solutions and emulsions are commercially available, for example under the trade names Masurf® FS-115 as a 14 wt. % solution of fluoroaliphatic phosphate ester in water, Masurf® FS-130 as a 28 wt. % solution of fluoroaliphatic phosphate ester in water, available from Mason Chemical Company. As is known in the art, siloxane surfactants are also commercially available.

The alcoholic composition of the present invention further includes at least one foam stabilizer. In one embodiment, where the foaming surfactant comprises a fluorosurfactant, the foam stabilizer may be selected from polymeric or oligomeric foam stabilizers. In another embodiment, where the foaming surfactant comprises a siloxane surfactant, the foam stabilizer may be polymeric or non-polymeric. In one embodiment, the foam stabilizer comprises a cationic oligomer or polymer.

Advantageously, the alcoholic composition of these embodiments exhibits improved foam stability when compared to an alcoholic composition without the foam stabilizer. By "foam stability" is meant the length of time that it takes for a foam to break down into a liquid.

Polymeric foam stabilizers include polyquaternium polymers. In general, a polyquaternium polymer is one that is designated as such by the CTFA. Polyquaternium polymers may be characterized by containing a quaternary ammonium group. Non-limiting examples of polyquaterniums include those listed in Table 1, below, including the INCI name and technical name.

**Table 1**

| **INCI Name Polyquaternium-X** | **Technical Name** |
|---|---|
| -2 | Bis(2-chloroethyl)ether, polym. w. N,N'-bis[3-(dimethylamino)propyl]urea |
| -4 | Hydroxyethylcellulose Dimethyldiallylammoinum Chloride Copolymer |
| -5 | Copolymer of acrylamide and beta-methacrylyloxyethyl trimethyl ammonium methosulfate |
| -6 | Polydimethyldiallyl Ammonium Chloride |
| -7 | Dimethyldiallyl Ammonium Chloride & Acrylamide Copolymer |
| -9 | Polydimethyaminoethyl methacrylate quaternized with Methyl Bromide |
| -10 | Hydroxyethylcellulose reacted with trimethyl ammonium substituted epoxide |
| -11 | PVP N,N-Dimethyl Aminoethyl Methacrylic Acid Copolymer Diethyl Sulfate Soln |
| -14 | Ethanaminium, N,N,N-Trimethyl-2-[(2-methyl-1-oxo-2-propenyl)oxy]-, Methyl Sulfate Homopolymer |
| -15 | Acrylamide-Dimethylaminoethyl Methacrylate Methyl Chloride Copolymer |
| -16 | 3-Methyl-1-Vinylimidazolium Chloride-1-Vinyl-2-Pyrrolidinone Chloride |
| -17 | Quat salt made from Adipic acid & diethylaminopropylamine & dichloroether |
| -18 | Quat salt prepared by the reaction of adipic acid and dimethylaminopropylamine, reacted with dichloroethyl ether |
| -19 | Quat ammonium salt prepared by the reaction of polyvinyl alcohol with 2,3- epoxypropylamine |
| -20 | Quat ammonium salt prepared by the reaction of polyvinyl octadecyl ether with 2,3-epoxypropylamine |
| -22 | Acrylic Acid-Diallyldimethylammonium Chloride (DADMAC) Polymer |
| -22 | Acrylic Acid-Diallyldimethylammonium Chloride (DADMAC) Polymer |
| -24 | Polyquat ammonium salt of hydroxyethyl cellulose reacted with lauryl dimethyl ammonium substituted epoxide |
| -27 | Block Copolymer of Polyquaternium-2 and 17 |
| -28 | Vinylpyrrolidone/Methacrylamidopropyltrimethylammonium Chloride Copolymer |
| -29 | Propoxylated Chitosan quaternized with epichlorhydrin |
| -30 | Ethanaminium, N-Carboxymethyl)-N,N-Dimethyl-2-((2-Methyl-1-Oxo-2-Propenyl)Oxy)-, Inner Salt, Polymer with Methyl 2-Methyl-2-Propenoate |
| -31 | 2-propane nitrile reaction product w/ N,N-dimethylpropanediamine, Sulfate |
| -32 | Acrylamide-Dimethylaminoethyl Methacrylate Methyl Chloride (DMAEMA) Copolymer |
| -37 | Trimethylaminoethyl Methacrylate Chloride Polymer |
| -39 | Acrylic Acid (AA), Polymer w/ Acrylamide & Diallyldimethylammonium Chloride(DADMAC) |
| -42 | Polyoxyethylene (dimethyliminio)ethylene-(dimethyliminio)ethylene dichloride |
| -43 | Copolymer of Acrylamide, acrylamidopropyltrimonium chloride, amidopropylacrylamide & DMAPA Monomers |
| -44 | Polyquat ammonium salt of vinylpyrrilidone & quaternized imidazoline monomers |
| -46 | Quat ammonium salt of vinylcaprolactum, vinylpyrrolidone &methylvinylimidazolium |
| -47 | Quat ammonium chloride- acrylic acid, methyl acrylate & methacrylamidopropyltrimonium Chloride |
| -48 | Copolymer of methacryolyl ethyl betaine, 2-hydroxyethylmethacrylate & methacryloylethyltrimethylammonium chloride |
| -51 | 3,5,8-Triox-4-Phosphaundec-10-en-1-aminium, 4-Hydroxy-N,N,N,10-Tetramethyl-9-Oxo, Inner Salt, 4-Oxide, Polymer with Butyl 2-Methyl-2-Propenoate |
| -53 | Acrylic Acid (AA)/Acrylamide/Methacrylamidopropyltrimonium Chloride (MAPTAC) Copolymer |
| -54 | Polymeric quaternary ammonium salt prepared by the reaction of aspartic acid and C6-18 alkylamine with dimethylaminopropylamine and sodium chloroacetate |
| -55 | 1-Dodecanaminium, N,N-Dimethyl-N-[3-[(2-Methyl-1-Oxo-2-Propenyl)AminoPropyl]-, Chloride, Polymer with N-[3-(Dimethylamino)Propyl]-2-Methyl-2-Propenamide and 1-Ethenyl-2-Pyrrolidinone |
| -56 | Polymeric quaternary ammonium salt prepared by the reaction of aspartic acid and C6-18 alkylamine with dimethylaminopropylamine and sodium chloroacetate. |
| -57 | Polymeric quaternary ammonium salt consisting of Castor Isostearate Succinate (q.v.) and Ricinoleamidopropyltrimonium Chloride (q.v.) monomers |
| -58 | 2-Propenoic Acid, Methyl Ester, Polymer with 2,2-Bis[(2-Propenyloxy)Methyl]-1-Butanol and Diethenylbenzene, Reaction Products with N,N-Dimethyl-1,3-Propanediamine, Chloromethane-Quaternized |
| -59 | Polyquaternium polyester |
| -60 | 9-Octadecenoic Acid, 12-Hydroxy-, [(2-Hydroxyethyl)Imino]Di-2,1-Ethanediyl Ester, Polymer with 5-Isocyanato-1-(Isocyanatomethyl)-1,3,3-Trimethylcyclohexane, Compd. with Diethyl Sulfate |
| -62 | Polymeric quaternary ammonium salt prepared by the reaction of butyl methacrylate, polyethylene glycol methyl ether methacrylate, ethylene glycol dimethacrylate and 2-methacryloyethyl trimonium chloride with 2,2'-azobis(2-methyl propionamidine) dihydrochloride |
| -63 | Copolymer of acrylamide, acrylic acid and ethyltrimonium chloride acrylate |
| -65 | Polymeric quaternary ammonium salt consisting of 2-methacryloyloxyethylphosphorylcholine, butyl methacrylate and sodium methacrylate monomers |
| -68 | Quaternized copolymers of vinylpyrrolidone (VP), methacrylamide(MAM) vinylimidazole(VI) & quaternized vinylimidazole (QVI) |

In one or more embodiments, the polyquaternium polymer includes a quaternized copolymer of vinylpyrrolidone and dimethylamino methacrylate, a hydrophobically modified quaternized copolymer of vinylpyrrolidone & dimethylaminopropyl methacrylamide, or a mixture thereof.

In one embodiment, the polyquaternium polymer has a molecular weight of from 1,000 to 5,000,000, in another embodiment, from about 1500 to about 2,500,000 and in yet another embodiment, from about 1,000,000 to about 2,000,000.

Other foam stabilizers that may operate to improve foam quality and/or stability include terpolymers of vinylcaprolactam (VCL), vinylpyrrolidone (VP) and dialkylaminoalkyl acrylate, including a VP/vinylcaprolactam/dimethylaminopropyl methacrylamide copolymer. Yet another foam stabilizer includes isobutylene/dimethylaminopropyl maleimide/ethoxylated maleimide/maleic acid copolymer. These and other foam stabilizers are sometimes referred to as film-forming polymers.

Still other foam stabilizers include acrylamide/ammonium acrylate copolymer, acrylamides/DMAPA acrylates/methoxy PEG methacrylate copolymer, acrylamide/sodium acryloyldimethyltaurate/acrylic acid copolymer, acrylamidopropyltrimonium chloride/acrylamide copolymer, acrylamidopropyltrimonium chloride/acrylates copolymer, acrylates/acetoacetoxyethyl methacrylate copolymer, acrylates/acrylamide copolymer, acrylates/ammonium methacrylate copolymer, acrylates/t-butylacrylamide copolymer, acrylates copolymer, acrylates/C1-2 succinates/hydroxyacrylates copolymer, acrylates/ethylamine oxide methacrylate copolymer, acrylates/lauryl acrylate/stearyl acrylate/ethylamine oxide methacrylate copolymer, acrylates/octylacrylamide copolymer, acrylates/octylacrylamide/diphenyl amodimethicone copolymer, acrylates/polytrimethyl siloxymethacrylate copolymer, acrylates/stearyl acrylate/ethylamine oxide methacrylate copolymer, acrylates/trifluoropropylmethacrylate/polytrimethyl siloxymethacrylate copolymer, acrylates/VA copolymer, acrylates/VP copolymer, adipic acid/diethylenetriamine copolymer, adipic acid/dimethylaminohydroxypropyl diethylenetriamine copolymer, adipic acid/epoxypropyl diethylenetriamine copolymer, adipic acid/isophthalic acid/neopentyl glycol/trimethylolpropane copolymer, allyl stearate/VA copolymer, aminoethylacrylate phosphate/acrylates copolymer, aminoethylpropanediol-acrylates/acrylamide copolymer, aminoethylpropanediol-AMPD-acrylates/diacetoneacrylamide copolymer, ammonium VA/acrylates copolymer, amodimethicone/silsesquioxane copolymer, AMPD-acrylates/diacetoneacrylamide copolymer, AMP-acrylates/allyl methacrylate copolymer, AMP-acrylates/Cl-18 alkyl acrylates/C1-8 alkyl acrylamide copolymer, AMP-acrylates/diacetoneacrylamide copolymer, AMP-acrylates/dimethylaminoethylmethacrylate copolymer, bacillus/rice bran extract/soybean extract ferment filtrate, behenyl methacrylate/ethylamine oxide methacrylate copolymer, bis-butyloxyamodimethicone/PEG-60 copolymer, bis-isobutyl PEG-14/amodimethicone copolymer, bis-isobutyl PEG-15/amodimethicone copolymer, butyl acrylate/ethylhexyl methacrylate copolymer, butyl acrylate/hydroxypropyl dimethicone acrylate copolymer, butyl ester of ethylene/MA copolymer, butyl ester of PVM/MA copolymer, calcium/sodium PVM/MA copolymer, chitosan, chitosan lactate, corn starch/acrylamide/sodium acrylate copolymer, dehydroxanthan gum, diethylene glycolamine/epichlorohydrin/piperazine copolymer, dimethicone crosspolymer, dimethicone/silsesquioxane copolymer, diphenyl amodimethicone, ethyl ester of PVM/MA copolymer, ethyltrimonium chloride methacrylate/hydroxyethylacrylamide copolymer, hydrolyzed wheat protein/PVP crosspolymer, hydroxypropyl dimethiconylpropyl acrylates copolymer, hydroxypropyltrimonium hydrolyzed corn starch, isobutylene/ethylmaleimide/hydroxyethylmaleimide copolymer, isobutylene/MA copolymer, isobutylmethacrylate/trifluoroethylmethacrylate/bis-hydroxypropyl dimethicone acrylate copolymer, isopropyl ester of PVM/MA copolymer, lauryl acrylate crosspolymer, lauryl methacrylate/glycol dimethacrylate crosspolymer, lauryl PEG-9 polydimethylsiloxyethyl dimethicone, methacrylic acid/sodium acrylamidomethyl propane sulfonate copolymer, methacryloyl ethyl betaine/acrylates copolymer, methoxy amodimethicone/silsesquioxane copolymer, methoxy PEG-114/polyepsilon caprolactone, myristic/palmitic/stearic/ricinoleic/eicosanedioic glycerides, octylacrylamide/acrylates/butylaminoethyl methacrylate copolymer, PEG-800/polyvinyl alcohol copolymer, PEG/PPG-25/25 dimethicone/acrylates copolymer, PEG-8/SMDI copolymer, polyacrylamide, polyacrylate-6, polyacrylate-8, polyacrylate-9, polyacrylate-15, polyacrylate-16, polyacrylate-17, polyacrylate-18, polyacrylate-19, polybeta-alanine/glutaric acid crosspolymer, polybutylene terephthalate, polyester-1, polyethylacrylate, polyethylene terephthalate, polyimide-1, polymethacryloyl ethyl betaine, polypentaerythrityl terephthalate, polyperfluoroperhydrophenanthrene, polyquatemium-4/hydroxypropyl starch copolymer, polyurethane-1, polyurethane-6, polyurethane-10, polyurethane-18, polyurethane-19, polyvinyl acetate, polyvinyl butyral, polyvinylcaprolactam, polyvinylformamide, polyvinyl imidazolinium acetate, polyvinyl methyl ether, potassium butyl ester of PVM/MA copolymer, potassium ethyl ester of PVM/MA copolymer, PPG-70 polyglyceryl-10 ether, PPG-12/SMDI copolymer, PPG-51/SMDI copolymer, PVM/MA copolymer, PVP/VA/itaconic acid copolymer, PVP/VA/vinyl propionate copolymer, rhizobian gum, rosin acrylate, shellac, silicone quatemium-16/glycidoxy dimethicone crosspolymer, sodium butyl ester of PVM/MA copolymer, sodium ethyl ester of PVM/MA copolymer, sodium polyacrylate, sodium polygamma-glutamate, soy protein phthalate, sterculia urens gum, terephthalic acid/Isophthalic acid/sodium isophthalic acid sulfonate/glycol copolymer, trimethylolpropane triacrylate, trimethylsiloxysilylcarbamoyl pullulan, VA/crotonates copolymer, VA/crotonates/methacryloxybenzophenone-1 copolymer, VA/crotonates/vinyl neodecanoate copolymer, VA/crotonates/vinyl propionate copolymer, VA/DBM copolymer, VA/vinyl butyl benzoate/crotonates copolymer, vinylamine/vinyl alcohol copolymer, vinyl caprolactam/VP/dimethylaminoethyl methacrylate copolymer, VP/acrylates/lauryl methacrylate copolymer, VP/dimethylaminoethylmethacrylate copolymer, VP/DMAPA acrylates copolymer, VP/hexadecene copolymer, VP/methacrylamide/vinyl imidazole copolymer, VP/VA copolymer, VP/vinyl caprolactam/DMAPA acrylates copolymer, yeast palmitate, a silicon-based polymer or resin such as phenylpropyldimethyl siloxysilicate, trimethylsiloxysilicate, cyclopentasiloxane, trimethylsiloxysilicate, diisostearoyl trimethyllolpropane siloxy silicate, vinyl dimethicone crosspoylmer/blends, and alkyl cetearyl dimethicone crosspolymers.

In one embodiment, the foam stabilizer includes a VP/vinylcaprolactam/dimethylaminopropyl methacrylamide copolymer sold under the trade names Aquaflex SF-40, or an isobutylene/dimethylaminopropyl maleimide/ethoxylated maleimide/maleic acid copolymer sold under the trade name Aquaflex XL-30.

In one embodiment, foam stabilizer is present in an amount of from about 0.005 to about 4 weight percent active, based upon the total weight of the alcoholic composition. In another embodiment, the foam stabilizer is present in an amount of from about 0.01 to about 1 weight percent, based upon the total weight of the alcoholic composition, and in yet another embodiment, the foam stabilizer is present in an amount of from about 0.02 to about 0.2 weight percent, based upon the total weight of the alcoholic composition.

In one embodiment, the foam stabilizer is added directly to the alcoholic composition. In one or more other embodiments, the foam stabilizer is added to the alcoholic composition as a solution or emulsion. In other words, the foam stabilizer may be premixed with a carrier to form a foam stabilizer solution or emulsion, with the proviso that the carrier does not deleteriously affect the foaming properties of the alcoholic composition. Examples of carriers include water, alcohol, glycols such as propylene or ethylene glycol, ketones, linear and/or cyclic hydrocarbons, triglycerides, carbonates, silicones, alkenes, esters such as acetates, benzoates, fatty esters, glyceryl esters, ethers, amides, polyethylene glycols and PEG/PPG copolymers, inorganic salt solutions such as saline, and mixtures thereof. It will be understood that, when the foam stabilizer is premixed to form a foam stabilizer solution or emulsion, the amount of solution or emulsion that is added to the alcoholic composition is selected so that the amount of foam stabilizer falls within the ranges set forth hereinabove.

In one embodiment, the weight ratio of foaming surfactant to foam stabilizer is from about 3.5:1 to about 14.5:1, and in another embodiment, the weight ratio of foaming surfactant to foam stabilizer is from about 8:1 to about 11:1.

In certain embodiments, the foam stabilizer increases the foam stability, i.e. the amount of time that it takes for foam to break down into a liquid. Particularly, it has been found that, when the foaming surfactant comprises a fluorosurfactant, the presence of a foam stabilizer improves the stability of the foam.

The foam stabilizer may operate to improve the stability of the foam in any number of ways. In one or more embodiments, the foam stabilizer also improves the foam quality, i.e. increases the number of bubbles and/or reduces the size of the bubbles.

Referring now to the drawings, FIG. 1A is a photograph showing a surface with a stabilized foamed composition on the right and an unstabilized foamed composition on the left, immediately after the foams were dispensed. More specifically, both foamed compositions contain the same amounts of ethanol, fluorosurfactant, humectant, and water. The foamed composition on the right also contains about 0.14 % by weight foam stabilizer. It can be seen that the unstabilized foam contains some small bubbles, but contains a greater amount of large, loose bubbles than the stabilized foam.

FIG. 1B is a photograph showing a surface with the foamed compositions of FIG. 1A, 1 minute after the foams were dispensed. It can be seen that, while the stabilized foam remain essentially unchanged, the unstabilized foam has become watery around the edges.

FIG. 1C is a photograph showing a surface with the foamed compositions of FIG. 1A, 3 minutes after the foams were dispensed. It can be seen that the stabilized foam remains essentially unchanged, while the unstabilized foam has become very watery.

FIG. 1D is a photograph showing a surface with the foamed compositions of FIG. 1A, 5 minutes after the foams were dispensed. It can be seen that the stabilized foam remains essentially unchanged, while the unstabilized foam is primarily liquid, with only a few bubbles remaining.

FIG. 1E is a photograph showing a surface with the foamed compositions of FIG. 1A, 1 hour after the foams were dispensed. It can be seen that, while the stabilized foam remains very foamy, the unstabilized foam has collapsed to become liquid. In embodiments, the stability of the foam produced when a composition comprising greater than 40 wt. % alcohol, a fluorosurfactant, and a foam stabilizer is passed through a non-aerosol foaming pump at room temperature if greater than the stability of the foam produced when a composition not including the foam stabilizer is passed through the non-aerosol foaming pump.

In one embodiment, the foam stability of the foam produced when the foamable alcoholic composition of the present invention is passed through a non-aerosol foaming pump at room temperature is at least about 30 seconds, in another embodiment at least about three minutes, or in other words, the alcoholic composition maintains its foam form and doesn't break down into liquid form for at least three minutes. In another embodiment, the foam stability is at least about five minutes, and in yet another embodiment, the foam stability is at least about 15 minutes. In one or more embodiments, the foam stability is at least about 30 minutes, and in other embodiments, the foam stability is at least about 60 minutes.

The alcoholic composition of this invention may further include a wide range of optional ingredients, with the proviso that they do not deleteriously affect the foam forming properties of the alcoholic composition, or the stability of the foam. The CTFA International Cosmetic Ingredient Dictionary and Handbook, Eleventh Edition, 2005, and the 2004 CTFA International Buyer's Guide describe a wide variety of non-limiting cosmetic and pharmaceutical ingredients commonly used in the skin care industry, that are suitable for use in the compositions of the present invention. Non-limiting examples of functional classes of ingredients are described in these references. Examples of these functional classes include: abrasives, anti-acne agents, anticaking agents, antioxidants, binders, biological additives, bulking agents, chelating agents, chemical additives; colorants, cosmetic astringents, cosmetic biocides, denaturants, drug astringents, emulsifiers, external analgesics, film formers, fragrance components, humectants, opacifying agents, plasticizers, preservatives, propellants, reducing agents, skin bleaching agents, skin-conditioning agents (emollient, humectants, miscellaneous, and occlusive), skin protectants, solvents, foam boosters, hydrotropes, solubilizing agents, suspending agents (nonsurfactant), sunscreen agents, ultraviolet light absorbers, detackifiers, and viscosity increasing agents (aqueous and nonaqueous). Examples of other functional classes of materials useful herein that are well known to one of ordinary skill in the art include solubilizing agents, sequestrants, and keratolytics, and the like. In one embodiment, the alcoholic composition further comprises glycerin.

Auxiliary surfactants may be included in the alcoholic compositions for the purpose of boosting or modifying the foam quality and characteristics, for modifying the feel of the final formulation during rub in and/or dry time, for providing persistence or long-lasting microbial action of the alcohol, for solubilizing other ingredients such as fragrances or sunscreens, and for irritation mitigation. Auxiliary surfactants include, but are not necessarily limited to, sulfosuccinates, amine oxides, PEG-80 sorbitan laurate, polyglucosides, alkanolamides, sorbitan derivatives, fatty alcohol ethoxylates, quaternary ammonium compounds, amidoamines, sultaines, isothionates, sarcosinates, betaines, and fatty alcohol polyethylene glycols.

Although a propellant may be used to produce stable foam, advantageously a propellant is not necessary. In certain embodiments, the amount of propellant is less than about 1000 parts per million by weight, based upon the total weight of the alcoholic composition. In one embodiment, the alcoholic composition is substantially free of propellants, such as hydrocarbon propellants. By substantially free is meant that the amount of propellant in the alcoholic composition is less than about 100 parts per million by weight, based upon the total weight of the alcoholic composition.

In one embodiment, alcohol is the only active antimicrobial ingredient introduced into the composition, and in this embodiment the amount of auxiliary antimicrobial ingredients is less than about 0.1 weight percent, based upon the total weight of the alcoholic composition. In other embodiments, the composition includes auxiliary antimicrobial agents in addition to alcohol. Examples of auxiliary antimicrobial agents include, but are not limited to, triclosan, also known as 5-chloro-2(2,4-dichlorophenoxy) phenol and available from Ciba-Geigy Corporation under the tradename IRGASAN®; chloroxylenol, also known as 4-chloro-3,5-xylenol, available from Nipa Laboratories, Inc. under the tradenames NIPACIDE® MX or PX; hexetidine, also known as 5-amino-1,3-bis(2-ethylhexyl)-5-methyl-hexahydropyrimidine; chlorhexidine salts including chlorhexidine gluconate and the salts of N,N"-Bis(4-chlorophenyl)-3,12-diimino-2,4,11,14-tetraazatetradecanediimidi amide; 2-bromo-2-nitropropane-1; 3-diol, benzalkonium chloride; cetylpyridinium chloride; alkylbenzyldimethylammonium chlorides; iodine; phenol derivatives, povidone-iodine including polyvinylpyrrolidinone-iodine; parabens; hydantoins and derivatives thereof, including 2,4-imidazolidinedione and derivatives of 2,4-imidazolidinedione as well as dimethylol-5,5-dimethylhydantoin (also known as DMDM hydantoin or glydant); phenoxyethanol; cis isomer of 1-(3-chloroallyl)-3,5,6-triaza-1-azoniaadamantane chloride, also known as quaternium-15 and available from Dow Chemical Company under the tradename DOWCIL^{™} 2000; diazolidinyl urea; benzethonium chloride; methylbenzethonium chloride; and mixtures thereof. When used, the auxiliary antimicrobial agents are present in amounts of from about 0.1 to about 1 wt. %, based upon the total weight of the alcoholic composition.

The alcoholic composition of the present invention may optionally further comprise a wide range of topical drug actives, with the proviso that they do not deleteriously affect the foam forming properties of the alcoholic composition, or the stability of the foam. Examples of topical drug actives include salicylic acid, acetyl salicylic acid, cis-retinoic acid, trans-retinoic acid, N-acetyl-L-cysteine, lipoic acid, azelaic acid, phytic acid, lisophosphotidic acid, tetracycline, ibuprofen, naproxen, acetominophen, hydrocortisone, resorcinol, phenoxyethanol, phenoxypropanol, phenoxyisopropanol, 2,4,4'-trichloro-2'-hydroxy diphenyl ether, 3,4,4'-trichlorocarbanilide, octopirox, 2-phenylbenzimidazole-5-sulfonic acid, dihydroxyacetone, benzoyl peroxide, 2,4,4'-trichloro-2-hydroxy diphenyl ether, phenoxyethanol, phenoxypropanol, phenoxyisopropanol, phytic acid, lipoic acid, lisophosphatidic acid, benoxaprofen, flubiprofen, fenoprofen, fenbufen, ketoprofen, indoprofen, priprofen, carprofen, oxaprozin, pranoprofen, miroprofen, tioxaprofen, suprofen, alminoprofen, tiaprofenic acid, fluprofen, bucloxic acid, benzocaine, lidocaine, bupivacaine, chloroprocaine, dibucaine, etidocaine, mepivacaine, tetracaine, dyclonine, hexylcaine, procaine, cocaine, ketamine, pramoxine, phenol, dihydroxyacetone, tyrosine, ethyltryosinate, phospho-DOPA, .beta.-lactim drugs, quinoline drugs, ciprofloxacin, norfloxacin, erythromycin, amikacin, doxycycline, capreomycin, chlorhexidine, chlortetracycline, oxytetracycline, clindamycin, ethambutol, hexamidinee isethionate, metronidazole, pentamidine, , gentamicin, kanamycin, lineomycin, methacyclin, methenamine, minocycine, neomycin, netilmicin, paromomycin, streptomycin, tobramycin, miconazole, tetracycline hydrochloride, erythromycin, zinc erythromycin, erythromycin estolate, erythromycin stearate, amikacin sulfate, doxyclcyline hydrochloride, capreomycin sulfate, chlorhexidine gluconate, chlorhexidine hydrochloride, chlortetracycline hydrochloride, oxytetracycline hydrochloride, clindamycin hydrochloride, ethambutol hydrochloride, metronidazole hydrochloride, pentamidine hydrochloride, gentamicin sulfate, kanamycin sulfate, lineomycin hydrochloride, methacycline hydrochloride, methenamine hippurate, methenamine mandelate, minocycline hydrochloride, neomycin sulfate, netilmicin sulfate, paromomycin sulfate, streptomycin sulfate, tobramycin sulfate, miconazole hydrochloride, amanfadine hydrochloride, amnanfadine sulfate, octopirox, parachlorometa xylenol, nystatin, tolnaftate, clotrimazole, 2-ethylhexyl p-methoxycinnamate, octyl methoxycinnamate, p-amino benzoate, p-aminobenzoic acid, 2-phenyl benzimidazole-5-sulfonic acid, octocrylene, oxybenzone, homomenthyl salicylate, octyl salicylate, 4,4'-methoxy-t-butyldibenzoylmethane, 4-isopropyl dibenzoylmethane, 3-benzylidene camphor, 3-(4-methylbenzylidene) camphor, titanium dioxide, silica, iron oxide, 4-N,N-(2-ethylhexyl)methyl aminobenzoic acid ester of 2,4-dihydroxybenzophenone, 4-N,N-(2-ethylhexyl)methyl aminobenzoic acid ester with 4-hydroxydibenzoylmethane, 4-N,N-(2-ethylhexyl)methyl aminobenzoic acid ester of 2-hydroxy-4-(2-hydroxyethoxy)benzophenone, 4-N,N-(2-ethylhexyl)-methyl aminobenzoic acid ester of 4-(2-hydroxyethoxy)dibenzoylmethane, tetracycline, ibuprofen, naproxen, acetaminophen, resorcinol, 3,4,4'-trichlorocarbanilide, octopirox, pharmaceutically-acceptable salts and mixtures of the above.

In one or more embodiments, the balance of the alcoholic composition includes water or other suitable solvent. In one embodiment, one or more volatile silicone-based materials are included in the formulation to further aid the evaporation process. Exemplary volatile silicones have a lower heat of evaporation than alcohol. In certain embodiments, use of silicone-based materials can lower the surface tension of the fluid composition. This provides greater contact with the surface. In one embodiment, the silicone-based material, such as cyclomethicone, trimethylsiloxy silicate or a combination thereof, may be included in the formulation at a concentration of from about 4 wt. % to about 50 wt. % and in another embodiment from about 5 wt. % to about 35 wt. %, and in yet another embodiment from about 11 wt. % to about 25 wt. %, based upon the total weight of the alcoholic composition.

In certain embodiments, such as the rinse-off formulation described above, the balance of the alcoholic composition includes foaming surfactant.

The alcoholic composition may be prepared by simply mixing the components together. The order of addition is not particularly limited. In one embodiment, the skin sanitizing alcoholic composition is prepared by a method comprising dispersing foaming surfactant in alcohol with slow to moderate agitation, adding water, and then adding a foam stabilizer, and mixing until the mixture is homogeneous.

The foamable composition of the present invention may be employed in any type of dispenser typically used for foam products. Advantageously, while the foamable composition can optionally be foamed by aerosolizing the composition, an aerosolized product is not necessary for foaming. Any dispenser that is capable of mixing the foamable alcoholic composition with air or an inert gas may be used. Inert gases include gas that does not substantially react or otherwise deleteriously affect the foamable composition. Examples of inert gases include nitrogen, argon, xenon, krypton, helium, neon, and radon. In one embodiment, the alcoholic composition is used in dispensers that employ foaming pumps, which combine ambient air or an inert gas and the alcoholic composition in a mixing chamber and pass the mixture through a mesh screen. In this and other embodiments, the viscosity of the composition is less than about 100 mPas, in one embodiment less than about 50 mPas, and in another embodiment less than about 25mPas.

The alcoholic composition of the present invention produces stabilized foam. As a result, the foamed composition will be less likely to break down to a liquid state and drip from the foaming pump and/or associated dispenser elements.

Accordingly, the present invention further provides a method of reducing dripping of a composition from a foaming pump. When a foamable composition is dispensed by using a foamable pump, a portion of the dispensed composition, now in foam form, remains in the dispenser head until another aliquot is dispensed. If the foam in the dispenser head breaks down into liquid between uses of the dispenser, the liquid will drip out of the dispenser head. The time between uses of the dispenser can be designated generally as X minutes. "X" can vary widely.

Advantageously, the stabilized foam of the present invention does not drip from the dispenser when X is about 3 minutes or less. In one embodiment, the stabilized foam does not drip from the dispenser when X is about five minutes or less, and in another embodiment, when X is about 15 minutes or less. In one or more embodiments, the stabilized foam does not drip from the dispenser when X is about 30 minutes or less, and in other embodiments, the stabilized foam does not drip from the dispenser when X is about 60 minutes or less.

In order to demonstrate the practice of the present invention, the following examples have been prepared and tested. The examples should not, however, be viewed as limiting the scope of the invention. The claims will serve to define the invention.

### Examples

Comparative Example 1 was prepared by dispersing the fluorosurfactant, DEA-C8-18 perfluoroalkylether phosphate, in ethanol with slow to moderate agitation until a homogeneous dispersion was achieved. Next, humectant was added and mixed until the mixture was homogeneous. Water was added, with mixing. The solution was agitated until a homogeneous mixture was achieved.

The foamable mixture was passed through an Airspray® foaming pump. The foam was rated for quality and stability as described hereinbelow, and the results are summarized in Table 2.

A simple 1-5 rating system was used to rate the foam of each system, with 5 being the best.

Foam Quality Rating Scale: Quality is a measurement of the visible appearance of the dispensed foam when dispensed from a typical commercial foaming pump.
Scale 0-5
0 No foam
1 Watery, weak foam with large loose bubbles
2 Large, loose bubbles, some small bubbles
3 Foam has about equal amounts of smaller and larger bubbles
4 Foam has primarily small, tight bubbles
5 Creamy, dense foam with small, tight bubbles

Foam Stability Rating Scale. Stability was rated by measuring the time it takes for the foam to breakdown into a liquid. A poor stability rating correlates to dripping from a wall or table top dispenser. Ratings of 3 or higher show considerable stabilization of the alcohol foam.
Scale 0 - 5
0 Immediately melts (<30 seconds)
1 Melts in about 30-60 seconds
2 Melts in about 1-15 minutes
3 Melts in about 15-30 minutes
4 Melts in about 30-60 minutes
5 Foam remains after about 60 minutes

Example 2 was prepared as described for Comparative Example 1, except that polyquaternium 11 was added after the water, and agitated until a homogeneous mixture was achieved. The foamable mixture was passed through a foaming pump and rated as for Comparative Example 1. The results are summarized in Table 2. The term "qs" indicates a sufficient amount to total 100 percent.

Comparative Example 3 and Examples 4-7 were prepared, foamed, and rated as described for Comparative Example 1 and Example 2, except that amounts were varied as shown in Table 2.

**Table 2**

| **Ingredient (weight percent)** | **Comments** | **Comparative Ex. 1** | **Ex. 2** | **Comparative Ex. 3** | **Ex. 4** | **Ex. 5** | **Ex. 6** | **Ex. 7** |
|---|---|---|---|---|---|---|---|---|
| Water | | qs | qs | qs | qs | qs | qs | qs |
| Ethanol | | 65.50 | 65.50 | 65.50 | 65.50 | 65.50 | 65.50 | 65.50 |
| DEA-C8-18 Perfluoroalkylethyl Phosphate | 15wt. % in water | 2.25 | 2.25 | 4.00 | 4.00 | 4.00 | 3.00 | 4.00 |
| Humectant | | 2.50 | 2.50 | 3.00 | 3.00 | 3.00 | 3.00 | 3.00 |
| Polyquaternium-11 | 20wt. % in water | 0 | 0.40 | 0 | 0.10 | 0.20 | 0.20 | 0.30 |
| Total | | 100 | 100 | 100 | 100 | 100 | 100 | 100 |
| | | | | | | | | |
| **Foam Quality Rating** | | 3 | 3 | 4 | 4 | 5 | 5 | 5 |
| **Foam Stability Rating** | | 2 | 5 | 2 | 3 | 5 | 5 | 5 |

Examples 8 - 11 were prepared as described for Example 2, except that in Examples 8, 9, and 11, polyquaternium 11 was replaced by other polymers. Each foamable mixture was passed through a foaming pump and rated as for Comparative Example 1. The results are summarized in Table 3.

**Table 3**

| **Ingredient (weight percent)** | **Comment** | **Ex. 8** | **Ex. 9** | **Ex. 10** | **Ex. 11** |
|---|---|---|---|---|---|
| **Water** | | qs | qs | qs | Qs |
| Ethanol | | 65.50 | 65.50 | 65.50 | 65.50 |
| DEA-C8-18 Perfluoroalkylethyl Phosphate | 15wt. % in water | 2.25 | 2.25 | 2.25 | 4.00 |
| Humectant | | 2.50 | 2.50 | 2.50 | 3.00 |
| Polyvinyl pyrrolidone | 95wt. % in water | 0.80 | -- | -- | -- |
| Isobutylene/dimethylaminopropyl maleimide/ethoxylated maleimide/maleic acid copolymer | 30wt. % in ethanol | -- | 0.10 | -- | -- |
| Quatemized copolymer of vinylpyrrolidone & dimethylaminopropyl methacrylamide (polyquaternium 11) | 20wt. % in water | -- | -- | 0.56 | -- |
| VP/Vinylcaprolactam/DMAPA Acrylates Copolymer | 40wt. % in ethanol | | | | 0.50 |
| **Total** | | 100 | 100 | 100 | 100 |
| | | | | | |
| **Foam Quality Rating** | | 3 | 3 | 4 | 4 |
| **Foam Stability Rating** | | 2 | 5 | 5 | 5 |

Examples 12-16, 19-21,23 and 24 and Comparative Examples 17, 18 and 22 were prepared as described for Example 1, except that the amounts of fluorosurfactant were varied, and two foaming surfactants, perfluorononylethyl carboxydecal peg10 dimethicone, and dimethicone copolymer undecylenate, were used in addition to or in place of the fluorosurfactant of Comparative Example 1. Example 10 also contains polyquaternium 11. Each foamable mixture was passed through a foaming pump and rated as for Comparative Example 1. The results are summarized in Tables 4 and 5.

**Table 4**

| **Ingredient** | **Comment** | **Ex. 12** | **Ex. 13** | **Ex. 14** | **Ex. 15** | **Ex. 16** | **Comparative Ex. 17** |
|---|---|---|---|---|---|---|---|
| Water | | 28.3 | 28.3 | 28.3 | 28.3 | 28.3 | 31.5 |
| Ethanol | | 65.5 | 65.5 | 65.5 | 65.5 | 65.5 | 65.5 |
| DEA C8-C18 perfluoroalkylethyl Phosphate | 15wt. % in water | 1.5 | 0.167 | 0.5 | 1.0 | 0.33 | 0.5 |
| Humectant | | 3 | 3 | 3 | 3 | 3 | -- |
| Perfluorononylethyl Carboxydecal PEG-10 Dimethicone | 100 | 1.5 | 2.83 | 2.5 | 2.0 | 2.67 | 2.5 |
| Polyquaternium 11 | 20wt. % in water | 0.2 | 0.2 | 0.2 | 0.2 | 0.2 | -- |
| | | | | | | | |
| | | | | | | | |
| **TOTAL** | | 100 | 100 | 100 | 100 | 100 | 100 |
| Foam quality rating | | 4 | 3 | 3 | 3 | 3 | 1 |
| Foam stability rating | | 3 | 2 | 3 | 3 | 2 | 2 |

**Table 5**

| **Ingredient** | **Comment** | **Comparative Ex. 18** | **Ex. 19** | **Ex. 20** | **Ex. 21** | **Comparative Ex. 22** | **Ex. 23** | **Ex. 24** |
|---|---|---|---|---|---|---|---|---|
| Water | | 31.5 | 30.3 | 31.8 | 33.3 | 31.5 | 31 | 31.5 |
| Ethanol | | 65.5 | 65.5 | 65.5 | 65.5 | 65.5 | 65.5 | 65.5 |
| DEA C8-C18 perfluoroalkylethyl Phosphate | 15wt. % in water | - | 3.0 | 1.0 | 0.5 | - | 1.0 | 0.75 |
| Humectant | | -- | -- | 1.0 | -- | -- | 1.0 | 1.0 |
| Perfluorononylethyl Carboxydecal PEG-10 Dimethicone | 100 | 3.0 | 1.0 | -- | -- | 3.0 | -- | -- |
| Dimethicone PEG-7 undecylenate | 100 | -- | -- | -- | -- | -- | 1.0 | 0.75 |
| Polyquaternium 11 | 20wt. % in water | -- | 0.2 | 0.7 | 0.7 | -- | 0.5 | 0.5 |
| | | | | | | | | |
| | | | | | | | | |
| **TOTAL** | | 100 | 100 | 100 | 100 | 100 | 100 | 100 |
| Foam quality rating | | 1 | 3 | 3 | 3 | 3 | 3 | 3 |
| Foam stability rating | | 2 | 4 | 3 | 3 | 2 | 4 | 3 |

Examples 25 -32 and 34- 36 and Comparative example 33 were prepared as described for Example 2, except that the amounts were varied as shown in Tables 6 and 7, and in some examples, various foaming surfactants were used in addition to or in place of the fluorosurfactant of Example 2. Comparative Example 33 does not contain polyquaternium 11. The results are summarized in Tables 6 and 7.

**Table 6**

| **Ingredient** | **Comment** | **Ex. 25** | **Ex. 26** | **Ex. 27** | **Ex. 28** | **Ex. 29** | **Ex. 30** |
|---|---|---|---|---|---|---|---|
| Water | | 28.2 | 29.3 | 28.2 | 28.2 | 28.2 | 29.2 |
| Ethanol | | 65.5 | 65.5 | 65.5 | 65.5 | 65.5 | 65.5 |
| DEA C8-C18 perfluotoalkylethyl Phosphate | 15wt. % in water | -- | 1 | 0.5 | 1 | 0.25 | -- |
| PEO-10 dimethicone | | 3 | 1 | 2.5 | 2 | 2.75 | 2 |
| Humectant | | 3 | 3 | 3 | 3 | 3 | 3 |
| Fragrance | | 0.075 | 0.075 | 0.075 | 0.075 | 0.075 | 0.075 |
| Polyquaternium 11 | 20wt. % in water | 0.2 | 0.2 | 0.2 | 0.2 | 0.2 | 0.2 |
| **TOTAL** | | 100 | 100 | 100 | 100 | 100 | 100 |
| Foam quality rating | | 4 | 5 | 5 | 5 | 4 | 3 |
| Foam stability rating | | 3 | 5 | 5 | 5 | 5 | 3 |

**Table 7**

| **Ingredient** | **Comment** | **Ex. 31** | **Ex.32** | **Comparative Ex. 33** | **Ex. 34** | **Ex. 35** | **Ex. 36** |
|---|---|---|---|---|---|---|---|
| Water | | 31.2 | 32.2 | 28.4 | 28.7 | 31 | 30.7 |
| Ethanol | | 65.5 | 65.5 | 65.5 | 65.5 | 65.5 | 65.5 |
| DEA C8-C18 perfluoroalkylethyl Phosphate | 15wt. % in water | -- | -- | -- | 0.5 | 0.25 | 0.5 |
| PEG-10 dimethicone | | 2 | 1 | 3 | 2 | 2 | 2 |
| Humectant | | 1 | 1 | 3 | 3 | 1 | 1 |
| Fragrance | | 0.075 | 0.075 | 0.075 | 0.075 | 0.075 | 0.075 |
| Polyquaternium 11 | 20wt. % in water | 0.2 | 0.2 | -- | 0.2 | 0.2 | 0.2 |
| **TOTAL** | | 100 | 100 | 100 | 100 | 100 | 100 |
| Foam quality rating | | 3 | 3 | 3 | 5 | 4 | 5 |
| Foam stability rating | | 3 | 3 | 3 | 5 | 5 | 5 |

The foamable composition of Comparative Example 1 was placed in an Airspray® foaming pump in a wall dispenser. An aliquot of the composition was dispensed from the pump. After about 30 seconds to 1 minute, liquid was observed dripping from the dispenser head.

To the foamable composition of Comparative Example 1 was added 0.1 wt. % polyqaternium 11. This composition was placed in an Airspray® foaming pump in a wall dispenser. An aliquot of the composition was dispensed from the pump. During about the next 10 minutes, no dripping was observed.

## Claims

1. A foamable composition comprising:
at least about 40 weight percent alcohol, based upon the total weight of the alcoholic composition; and
a foaming surfactant selected from the group consisting of siloxane polymer surfactants, anionic, cationic, and zwitterionic fluorosurfactants, and combinations thereof; and
a polymeric or oligomeric cationic foam stabilizer.

2. A method for reducing dripping of a composition from a foaming pump dispenser adapted to dispense said composition, the method comprising:
providing a foamable composition comprising at least about 40 weight percent alcohol, based upon the total weight of the alcoholic composition; a foaming surfactant selected from the group consisting of siloxane polymer surfactants, anionic, cationic, and zwitterionic fluorosurfactants, and combinations thereof; and a polymeric or oligomeric foam stabilizer;
providing a foaming pump dispenser having a dispenser head;
placing said foamable composition into said foaming pump dispenser; and
dispensing an aliquot of said foamable composition, wherein a portion of said aliquot remains in the dispenser head for at least about 3 minutes, and wherein said portion remaining in said dispenser head does not drip from the dispenser.

3. A method for stabilizing a foam formed from a foamable alcoholic composition, the method comprising:
providing an alcoholic composition comprising an alcohol, and a foaming surfactant; and
combining the alcoholic composition with a polymeric or oligomeric cationic foam stabilizer to form a stabilized foam, wherein the foam stability of the stabilized foam is greater than the stability of a foam that does not contain a foam stabilizer, and wherein the foam stability of said stabilized foam at room temperature is at least about 30 seconds.

4. The composition of claim 1, or method of claim 2 or 3, wherein said foaming surfactant comprises a fluorosurfactant compound that may be represented by the formula
[F₃CF₂C - (CF₂CF₂)ₓ -CH₂CH₂-O-P₂O₃] - [ R¹] ⁺
where [R¹] + includes DEA, TEA, NH4, or betaine, and where x is an integer from about 4 to about 18.

5. The composition of claim 1, or method of claim 2 or 3, wherein said foaming surfactant comprises a siloxane polymer surfactant that may be represented by the formula
R² - Si (CH₃)₂ - [O-Si (CH₃)₂]ₐ - [O - Si (CH₃) R³]_{b} - O - Si (CH₃)₂ - R²
where R² and R³ independently include a methyl group or a moiety that may be represented by the formula
-(CH₂)₃ - O - (CH₂CH₂O)_{c} - [CH₂CH(CH₃)O]_{d} - (CH₂CH₂O)ₑ H
with the proviso that both R² and R³ are not CH₃, where a is an integer from about 3 to about 21, b is an integer from about 1 to about 7, c is an integer from about 0 to about 40, d is an integer from about 0 to about 40, and e is an integer from about 0 to about 40, with the proviso that a ≥ 3 x b and that c + d + e ≥ 5.

6. The composition of claim 1, or method of claim 2 or 3, wherein said siloxane polymer surfactant comprises a surfactant selected from the group consisting of organopolysiloxane dimethicone polyols, silicone carbinol fluids, silicone polyethers, alkylmethyl siloxanes, amodimethicones, trisiloxane ethoxylates, dimethiconols, quaternized silicone surfactants, polysilicones, silicone crosspolymers, silicone waxes, and mixtures thereof.

7. The composition of claim 1, or method of claim 2 or 3, wherein said siloxane polymer surfactant comprises dimethicone PEG-7 undecylenate, PEG-10 dimethicone, PEG-8 dimethicone, PEG-12 dimethicone, perfluorononylethyl carboxydecal PEG 10, PEG-20/PPG-23 dimethicone, PEG-11 methyl ether dimethicone, bis-PEG/PPG-20/20 dimethicone, silicone quats, PEG-9 dimethicone, PPG-12 dimethicone, fluoro PEG-8 dimethicone, PEG 23/PPG 6 dimethicone, PEG 20/PPG 23 dimethicone, PEG 17 dimethicone, PEG5/PPG3 methicone, bis-PEG20 dimethicone, a PEG/PPG20/15 dimethicone copolyol and sulfosuccinate blend, or a mixture thereof.

8. The composition of claim 1, or method of claim 2 or 3, wherein said foam stabilizer comprises a polyquaternium, a quaternized copolymer of vinylpyrrolidone and dimethylamino methacrylate, a hydrophobically modified quaternized copolymer of vinylpyrrolidone & dimethylaminopropyl methacrylamide, or a mixture thereof.

9. The composition of claim 1, or method of claim 2 or 3, wherein said foam stabilizer comprises a terpolymer of vinylcaprolactam (VCL), vinylpyrrolidone (VP) and dialkylaminoalkyl acrylate, or a isobutylene/dimethylaminopropyl maleimide/ethoxylated maleimide/maleic acid copolymer.

10. An antimicrobial composition comprising:
at least about 50 weight percent alcohol, based upon the total weight of the alcoholic composition;
from about 0.002 to about 4 wt. % of a surfactant comprising a fluorosurfactant selected from the group consisting of compounds represented by the formula
[F₃CF₂C - (CF₂CF₂)ₓ -CH₂CH₂-O-P₂O₃] - [ R¹] ⁺
where [R¹] + includes DEA, TEA, NH4, or betaine, and where x is an integer from about 4 to about 18, or a siloxane polymer surfactant, selected from the group consisting of compounds represented by the formula
R² - Si (CH₃)₂ - [O-Si (CH₃)₂]ₐ - [O - Si (CH₃) R³ ]_{b} - O - Si (CH₃)₂ - R²
where R² and R³ independently include a methyl group or a moiety that may be represented by the formula
-(CH₂), - O - (CH₂CH₂O)_{c} - [CH₂CH(CH₃)O]_{d} - (CH₂CH₂O)ₑ H
with the proviso that both R² and R³ are not CH₃, where a is an integer from about 3 to about 21, b is an integer from about 1 to about 7, c is an integer from about 0 to about 40, d is an integer from about 0 to about 40, and e is an integer from about 0 to about 40, with the proviso that a ≥ 3 x b and that c + d + e ≥ 5, or a combination thereof; and
from about 0.005 to about 4 wt. % of a polymeric or oligomeric cationic foam stabilizer.

11. A foamable composition comprising:
at least about 40 weight percent alcohol, based upon the total weight of the alcoholic composition; and
a foaming surfactant selected from the group consisting of siloxane polymer surfactants, anionic, cationic, and zwitterionic fluorosurfactants, and combinations thereof; and
a polymeric or oligomeric foam stabilizer selected from the group consisting of polyquaternium polymers, terpolymers of vinylcaprolactam (VCL), vinylpyrrolidone (VP) and dialkylaminoalkyl acrylate, isobutylene/dimethylaminopropyl maleimide/ethoxylated maleimide/maleic acid copolymer, acrylamide/ammonium acrylate copolymer, acrylamides/DMAPA acrylates/methoxy PEG methacrylate copolymer, acrylamide/sodium acryloyldimethyltaurate/acrylic acid copolymer, acrylamidopropyltrimonium chloride/acrylamide copolymer, acrylamidopropyltrimonium chloride/acrylates copolymer, acrylates/acetoacetoxyethyl methacrylate copolymer, acrylates/acrylamide copolymer, acrylates/ammonium methacrylate copolymer, acrylates/t-butylacrylamide copolymer, acrylates copolymer, acrylates/C1-2 succinates/hydroxyacrylates copolymer, acrylates/ethylamine oxide methacrylate copolymer, acrylates/lauryl acrylate/stearyl acrylate/ethylamine oxide methacrylate copolymer, acrylates/octylacrylamide copolymer, acrylates/octylacrylamide/diphenyl amodimethicone copolymer, acrylates/polytrimethyl siloxymethacrylate copolymer, acrylates/stearyl acrylate/ethylamine oxide methacrylate copolymer, acrylates/trifluoropropylmethacrylate/polytrimethyl siloxymethacrylate copolymer, acrylates/VA copolymer, acrylates/VP copolymer, adipic acid/diethylenetriamine copolymer, adipic acid/dimethylaminohydroxypropyl diethylenetriamine copolymer, adipic acid/epoxypropyl diethylenetriamine copolymer, adipic acid/isophthalic acid/neopentyl glycol/trimethylolpropane copolymer, allyl stearate/VA copolymer, aminoethylacrylate phosphate/acrylates copolymer, aminoethylpropanediol-acrylates/acrylamide copolymer, aminoethylpropanediol-AMPD-acrylates/diacetoneacrylamide copolymer, ammonium VA/acrylates copolymer, amodimethicone/silsesquioxane copolymer, AMPD-acrylates/diacetoneacrylamide copolymer, AMP-acrylates/allyl methacrylate copolymer, AMP-acrylates/C1-18 alkyl acrylates/C1-8 alkyl acrylamide copolymer, AMP-acrylates/diacetoneacrylamide copolymer, AMP-acrylates/dimethylaminoethylmethacrylate copolymer, bacillus/rice bran extract/soybean extract ferment filtrate, behenyl methacrylate/ethylamine oxide methacrylate copolymer, bis-butyloxyamodimethicone/PEG-60 copolymer, bis-isobutyl PEG-14/amodimethicone copolymer, bis-isobutyl PEG-15/amodimethicone copolymer, butyl acrylate/ethylhexyl methacrylate copolymer, butyl acrylate/hydroxypropyl dimethicone acrylate copolymer, butyl ester of ethylene/MA copolymer, butyl ester of PVM/MA copolymer, calcium/sodium PVM/MA copolymer, chitosan lactate, corn starch/acrylamide/sodium acrylate copolymer, diethylene glycolaminelepichlorohydrin/piperazine copolymer, dimethicone crosspolymer, dimethicone/silsesquioxane copolymer, diphenyl amodimethicone, ethyl ester of PVM/MA copolymer, ethyltrimonium chloride methacrylate/hydroxyethylacrylamide copolymer, hydrolyzed wheat protein/PVP crosspolymer, hydroxypropyl dimethiconylpropyl acrylates copolymer, hydroxypropyltrimonium hydrolyzed corn starch, isobutylene/ethylmaleimide/hydroxyethylmaleimide copolymer, isobutylene/MA copolymer, isobutylmethacrylate/trifluoroethylmethacrylate/bis-hydroxypropyl dimethicone acrylate copolymer, isopropyl ester of PVM/MA copolymer, lauryl acrylate crosspolymer, lauryl methacrylate/glycol dimethacrylate crosspolymer, lauryl PEG-9 polydimethylsiloxyethyl dimethicone, methacrylic acid/sodium acrylamidomethyl propane sulfonate copolymer, methacryloyl ethyl betaine/acrylates copolymer, methoxy amodimethicone/silsesquioxane copolymer, methoxy PEG-114/polyepsilon caprolactone, myristic/palmitic/stearic/ricinoleic/eicosanedioic glycerides, octylacrylamide/acrylates/butylaminoethyl methacrylate copolymer, PEG-800/polyvinyl alcohol copolymer, PEG/PPG-25/25 dimethicone/acrylates copolymer, PEG-8/SMDI copolymer, polyacrylamide, polyacrylate-6, polyacrylate-8, polyacrylate-9, polyacrylate-15, polyacrylate-16, polyacrylate-17, polyacrylate-18, polyacrylate-19, polybeta-alanine/glutaric acid crosspolymer, polybutylene terephthalate, polyester-1, polyethylacrylate, polyethylene terephthalate, polyimide-1, polymethacryloyl ethyl betaine, polypentaerythrityl terephthalate, polyperfluoroperhydrophenanthrene, polyquaternium-4/hydroxypropyl starch copolymer, polyurethane-1, polyurethane-6, polyurethane-10, polyurethane-18, polyurethane-19, polyvinyl acetate, polyvinyl butyral, polyvinylcaprolactam, polyvinylformamide, polyvinyl imidazolinium acetate, potassium butyl ester of PVM/MA copolymer, potassium ethyl ester of PVM/MA copolymer, PPG-12/SMDI copolymer, PPG-51/SMDI copolymer, PVM/MA copolymer, PVP/VA/itaconic acid copolymer, PVP/VA/vinyl propionate copolymer, rhizobian gum, rosin acrylate, shellac, silicone quaternium-16/glycidoxy dimethicone crosspolymer, sodium butyl ester of PVM/MA copolymer, sodium ethyl ester of PVM/MA copolymer, sodium polyacrylate, sodium polygamma-glutamate, soy protein phthalate, sterculia urens gum, terephthalic acid/Isophthalic acid/sodium isophthalic acid sulfonate/glycol copolymer, trimethylolpropane triacrylate, trimethylsiloxysilylcarbamoyl pullulan, VA/crotonates copolymer, VA/crotonates/methacryloxybenzophenone-1 copolymer, VA/crotonates/vinyl neodecanoate copolymer, VA/crotonates/vinyl propionate copolymer, VA/DBM copolymer, VA/vinyl butyl benzoate/crotonates copolymer, vinylamine/vinyl alcohol copolymer, vinyl caprolactam/VP/dimethylaminoethyl methacrylate copolymer, VP/acrylates/lauryl methacrylate copolymer, VP/dimethylaminoethylmethacrylate copolymer, VP/DMAPA acrylates copolymer, VP/hexadecene copolymer, VP/methacrylamide/vinyl imidazole copolymer, VP/VA copolymer, VP/vinyl caprolactam/DMAPA acrylates copolymer, yeast palmitate, a silicon-based polymer or resin such as phenylpropyldimethyl siloxysilicate, trimethylsiloxysilicate, cyclopentasiloxane, trimethylsiloxysilicate, diisostearoyl trimethyllolpropane siloxy silicate, vinyl dimethicone crosspolymer/blends, alkyl cetearyl dimethicone crosspolymers, and mixtures thereof.

## Patentansprüche

1. Schäumbare Zusammensetzung umfassend:
mindestens ca. 40 Gewichtsprozent Alkohol, bezogen auf das Gesamtgewicht der alkoholischen Zusammensetzung; und
ein schaumbildendes Tensid ausgewählt aus der Gruppe bestehend aus Siloxan-Polymer-Tensiden, anionischen, kationischen und zwitterionischen Fluortensiden, sowie Kombinationen davon; und
einem polymeren oder oligomeren Schaumstabilisator.

2. Methode zur Verringerung des Tropfens einer Zusammensetzung aus einem Schaumspender, welcher zur Abgabe der Zusammensetzung ausgelegt ist, wobei die Methode folgendes umfasst:
Bereitstellung einer schäumbaren Zusammensetzung umfassend mindestens ca. 40 Gewichtsprozent Alkohol, bezogen auf das Gesamtgewicht der alkoholischen Zusammensetzung; eines schaumbildenden Tensids ausgewählt aus der Gruppe bestehend aus Siloxan-Polymer-Tensiden, anionischen, kationischen und zwitterionischen Fluortensiden, sowie Kombinationen davon; und eines polymeren oder oligomeren Schaumstabilisators;
Bereitstellung eines Schaumspenders mit einem Spenderkopf;
Einbringung der schäumbaren Zusammensetzung in den Schaumspender; und
Abgabe eines Aliquots der schäumbaren Zusammensetzung, wobei ein Teil des Aliquots mindestens 3 Minuten lang im Spenderkopf verbleibt, und wobei die im Spenderkopf verbleibende Zusammensetzung nicht aus dem Spender tropft .

3. Methode zur Stabilisierung eines aus einer schäumbaren alkoholischen Zusammensetzung gebildeten Schaumes, wobei die Methode folgendes umfasst:
Bereitstellung einer alkoholischen Zusammensetzung, welche einen Alkohol und ein schaumbildendes Tensid umfasst; und
Kombination der alkoholischen Zusammensetzung mit einem kationischen polymeren oder oligomeren Schaumstabilisator zu einem stabilisierten Schaum, wobei die Stabilität des stabilisierten Schaums größer ist als die Stabilität eines Schaumes ohne Schaumstabilisator, und wobei die Schaumstabilität des genannten stabilisierten Schaumes bei Raumtemperatur mindestens ca. 30 Sekunden vorhält.

4. Zusammensetzung von Anspruch 1, oder die Methode von Anspruch 2 oder 3, wobei das Schaumtensid eine zusammengesetzte Fluortensidverbindung umfasst, welche durch folgende Formel repräsentiert werden kann
[F₃CF₂C - (CF₂CF₂)x -CH₂CH₂-O-P₂O₃]- [R¹] +
wobei [R¹] + DEA, TEA, NH4 oder Betain einschließt und wobei x eine ganze Zahl von etwa 4 bis etwa 18 ist.

5. Zusammensetzung von Anspruch 1, oder die Methode von Anspruch 2 oder 3, wobei das Schaumtensid ein zusammengesetztes Siloxan-Polymertensid umfasst, welches durch folgende Formel repräsentiert werden kann
R² - Si (CH₃)₂ - [O-Si (CH₃)₂]ₐ - [O - Si (CH₃) R₃]_{b} - O - Si (CH₃)₂ - R²
wobei R² und R³ unabhängig voneinander eine Methylgruppe oder eine Seitenkette sind, welche durch folgende Formel repräsentiert werden kann
-(CH₂)₃ - O - (CH₂CH₂O)_{c} - [CH₂CH(CH₃)O]_{d} - (CH₂CH₂O)ₑ H
mit der Maßgabe, dass R² und R³ nicht beide CH₃ sind , wobei a eine ganze Zahl von etwa 3 bis etwa 21 ist, b eine ganze Zahl von etwa 1 bis etwa 7 ist, c eine ganze Zahl von 0 bis etwa 40 ist , d eine ganze Zahl von etwa 0 bis etwa 40 ist, und e eine ganze Zahl von 0 bis zu etwa 40 ist, mit der Maßgabe, dass a ≥ 3 x b und c + d + e ≥ 5.

6. Zusammensetzung von Anspruch 1, oder die Methode von Anspruch 2 oder 3, wobei das Siloxan-Polymer-Tensid ein Tensid umfasst, ausgewählt aus der Gruppe bestehend aus Organopolysiloxan-Dimethiconpolyolen, Silikoncarbinol-Flüssigkeiten, Silikon-Polyethern, Alkylmethylsiloxanen, Amodimethiconen, Trisiloxanethoxylatderivaten, Dimethiconolen, quaternisierten Silikon-Tensiden, Polysilikonen, Silikon-Kreuzpolymeren , Silikonwachsen und Gemischen derselben.

7. Zusammensetzung von Anspruch 1, oder die Methode von Anspruch 2 oder 3, wobei das Siloxan-Polymertensid Dimethicon PEG-7 Undecylenat, PEG-10 Dimethicon, PEG-8 Dimethicon, PEG-12 Dimethicon, Perfluornonylethylcarboxydecal PEG-10, PEG-20/PPG-23 Dimethicon, PEG-11 Methyletherdimethicon, Bis-PEG/PPG-20/20 Dimethicon, Silikon-QAV, PEG-9 Dimethicon, PPG-12 Dimethicon, Fluor-PEG-8 Dimethicon, PEG 23/PPG 6 Dimethicon, PEG 20/PPG 23 Dimethicon, PEG-17 Dimethicon, PEG5/PPG3 Methicon, Bis-PEG20 Dimethicon, ein PEG/PPG20/15 Dimethiconcopolyol und - sulfosuccinatgemisch, oder eine Gemisch derselben umfasst.

8. Zusammensetzung von Anspruch 1, oder die Methode von Anspruch 2 oder 3, wobei der Schaumstabilisator ein Polyquaternium, ein quaternisiertes Copolymer aus Vinylpyrrolidon und Dimethylaminomethacrylat, ein hydrophob modifiziertes quaternisiertes Vinylpyrrolidon-Copolymer und Dimethylaminopropylmethacrylamid, oder ein Gemisch derselben umfasst.

9. Zusammensetzung von Anspruch 1, oder die Methode von Anspruch 2 oder 3, wobei der Schaumstabilisator ein Terpolymer von Vinylcaprolactam (VCL), Vinylpyrrolidon (VP) und Dialkylaminoalkylacrylat oder ein Isobutylen-/Dimethylaminopropylmaleimid-/ethoxyliertes Maleimid-/Maleinsäure-Copolymer umfasst.

10. Antimikrobielle Zusammensetzung, umfassend:
mindestens ca. 50 Gewichtsprozent Alkohol, bezogen auf das Gesamtgewicht der alkoholischen Zusammensetzung; und
von etwa 0,002 bis etwa 4 Gew. % eines Tensids, welches ein Fluortensid umfasst, ausgewählt aus der Gruppe bestehend aus den durch die folgende Formel repräsentierten Verbindungen
[F₃CF₂C - (CF₂CF₂)ₓ -CH₂CH₂-O-P₂O₃]- [R¹] +
wobei [R¹] + DEA, TEA, NH₄ oder Betain einschließt, und wobei x eine ganze Zahl von etwa 4 bis etwa 18 ist, oder ein Siloxanpolymer-Tensid, ausgewählt aus der Gruppe bestehend aus durch die folgende Formel repräsentierten Verbindungen
R² - Si (CH₃)₂ - [O-Si (CH₃)₂]ₐ - [O - Si (CH₃) R₃]_{b}- O - Si (CH₃)₂ - R²
wobei R² und R³ unabhängig voneinander eine Methylgruppe oder eine Seitenkette einschließen, welche durch folgende Formel repräsentiert werden kann
-(CH₂)₃ - O - (CH₂CH₂O)_{c} - [CH₂CH(CH₃)O]_{d} - (CH₂CH₂O)ₑ H
mit der Maßgabe, dass R² und R³ nicht beide CH₃ sind , wobei a eine ganze Zahl von etwa 3 bis etwa 21 ist, b eine ganze Zahl von etwa 1 bis etwa 7 ist, c eine ganze Zahl von 0 bis etwa 40 ist, d eine ganze Zahl von etwa 0 bis etwa 40 ist, und e eine ganze Zahl von 0 bis etwa 40 ist, mit der Maßgabe, dass a ≥ 3 x b und c + d + e ≥ 5, oder eine Kombination derselben ist; und
von ca. 0,005 bis ca. 4 Gew. % eines polymeren oder oligomeren Schaumstabilisators.

11. Schäumbare Zusammensetzung umfassend:
mindestens ca. 40 Gewichtsprozent Alkohol, bezogen auf das Gesamtgewicht der alkoholischen Zusammensetzung; und
ein schäumendes Tensid ausgewählt aus der Gruppe bestehend aus Siloxan-Polymer-Tensiden, anionischen, kationischen und zwitterionischen Fluortensiden, sowie Kombinationen davon; und
einen polymeren oder oligomeren Schaumstabilisator ausgewählt aus der Gruppe bestehend aus Polyquaternium-Polymeren, Terpolymeren von Vinylcaprolactam (VCL), Vinylpyrrolidon (VP) und Dialkylaminoalkylacrylat , Isobutylen / Dimethylaminopropylmaleimid /ethoxyliertem Maleimid / Maleinsäure-Copolymer, Acrylamid / Ammoniumacrylat-Copolymer, Acrylamiden / DMAPA Acrylaten / Methoxy PEG-Methacrylat-Copolymer, Acrylamid / Natriumacryloyldimethyltaurat / AcrylsäureCopolymer, Acrylamidpropyltrimoniumchlorid / Acrylamid-Copolymer, Acrylamidpropyltrimoniumchlorid / Acrylate-Copolymer, Acrylate / Acetoacetoxyethylmethacrylat - Copolymer, Acrylate / Acrylamid-Copolymer, Acrylate / Ammoniummethacrylat-Copolymer, Acrylate / T-Butylacrylamid-Copolymer, Acrylate -Copolymer, Acrylate /C1-2 Succinate / Hydroxyacrylate-Copolymer, Acrylate / Ethylaminoxid Methacrylat -Copolymer, Acrylate / Laurylacrylat / Stearylacrylat / Ethylaminoxid-Methacrylat-Copolymer, Acrylate / Octylacrylamid-Copolymer, Acrylate / Octylacrylamid / Diphenylamodimethicon-Copolymer, Acrylate / Polytrimethylsiloxymethacrylat-Copolymer, Acrylate / Stearylacrylat / Ethylaminoxid-Methacrylat-Copolymer, Acrylate / Trifluoropropylmethacrylat / Polytrimethyl-Siloxymethacrylat-Copolymer, Acrylate /VA-Copolymer, Acrylate /VP-Copolymer, Adipinsäure/ Diethylentriamin-Copolymer, Adipinsäure/ Dimethylaminohydroxypropyldiethylentriamin-Copolymer, Adipinsäure / Epoxypropyldiethylentriamin-Copolymer, Adipinsäure / Isophthalsäure / Neopentylglykol / Trimethylolpropan-Copolymer, Allylstearat /VA-Copolymer, Aminoethylacrylatphosphat / Acrylate -Copolymer, Aminoethylpropanediol-Acrylate /Acrylamid-Copolymer, Aminoethylpropandiol-AMPD-Acrylate /Diacetonacrylamid-Copolymer, Ammonium VA / Acrylate -Copolymer, Amodimethicon / Silsesquioxan-Copolymer, AMPD-Acrylate /Diacetonacrylamid- Copolymer, AMP-Acrylate /Allylmethacrylat - Copolymer, AMP-Acrylate /C1-18 Alkylacrylate /C1-8 Alkylacrylamid-Copolymer, AMP-Acrylate /Diacetonacrylamid-Copolymer, AMP-Acrylate/Dimethylaminoethylmethacrylat- Copolymer, Bacillus / Reis Kleie-Extrakt / Filtrat von fermentiertem Sojabohnenextrakt , Behenylmethacrylat / Ethylaminoxidmethacrylat-Copolymer, Bis-Butyloxyamodimethicon/PEG-60 Copolymer, Bis-Isobutyl PEG-14/Amodimethicon Copolymer, Bis-Isobutyl PEG-15/Amodimethicon-Copolymer, Butylacrylat / Ethylhexyl-Methacrylat-Copolymer, Butylacrylat / Hydroxypropyldimethicon-Acrylat-Copolymer, Butylester von Ethylen/ MA-Copolymer, Butylester von PVM/MA-Copolymer, Calcium-/ Natrium-PVM/MA-Copolymer, Chitosan-Laktat, Maisstärke / Acrylamid / Natriumacrylat-Copolymer, Diethylenglykolamin/ Epichlorhydrin / Piperazin-Copolymer, Dimethicon-Kreuzpolymer, Dimethicon / Silsesquioxan-Copolymer, Diphenylamodimethicon, Ethylester vom PVM/MA-Copolymer, Ethyltrimoniumchloridmethacrylat/Hydroxyethylacrylamid-Copolymer, hydrolysiertes Weizenprotein / PVP-Kreuzpolymer, Hydroxypropyldimethiconylpropylacrylate-Copolymer, Hydroxypropyltrimonium hydrolysierte Maisstärke, Isobutylen / Ethylmaleimid / Hydroxyethylmaleimid-Copolymer, Isobutylen/MA-Copolymer, Isobutylmethacrylat/ Trifluorethylmethacrylat / Bis-Hydroxypropyldimethiconacrylat-Copolymer, Isopropylester vom PVM/MA Copolymer, Laurylacrylat-Kreuzpolymer, Laurylmethacrylat / Glykoldimethacrylat-Kreuzpolymer, Lauryl-PEG-9 Polydimethylsiloxyethyldimethicon, Methacrylsäure-/Natriumacrylamidmethylpropansulfonat-Copolymer, Methacryloylethylbetain / Acrylate-Copolymer, Methoxyamodimethicon-/ Silsesquioxan-Copolymer, Methoxy PEG-114-/Polyepsilon-Caprolacton, Myristat / Palmitat / Stearat / Ricinolen / Eicosandion-Glyceride, Octylacrylamid / Acrylate / Butylaminoethylmethacrylat-Copolymer, PEG-800/Polyvinylalkohol-Copolymer, PEG/PPG-25/25 Dimethicon / Acrylate-Copolymer, PEG-8/SMDI-Copolymer, Polyacrylamid, Polyacrylat-6, Polyacrylat-8, Polyacrylat-9, Polyacrylat-15, Polyacrylat-16, Polyacrylat-17, Polyacrylat-18, Polyacrylat-19, Polybetaalanin/Glutarsäure-Kreuzpolymer, Polybutylenterephthalat, Polyester-1, Polyethylacrylat, Polyethylenterephthalat, Polyimid-1, Polymethacryloylethyl-Betain, Polypentaerythritylterephthalat, Polyperfluorperhydrophenanthren, Polyquaternium-4/Hydroxypropyl-Stärke-Copolymer, Polyurethan-1, Polyurethan-6, Polyurethan-10, Polyurethan-18, Polyurethan-19, Polyvinylacetat, Polyvinylbutyral, Polyvinylcaprolactam, Polyvinylformamid, Polyvinyl-Imidazoliniumacetat, Kaliumbutylester vom PVM/MA-Copolymer, Kaliumethylestervom PVM/MA-Copolymer, PPG-12/SMDI-Copolymer, PPG-51/SMDI-Copolymer, PVM/MA-Copolymer , PVP/VA/Itaconsäure-Copolymer, PVP / VA/Vinylpropionat-Copolymer, Rhizobian-Gummi, Rosin-Acrylat, Schellack, Silikon Quaternium-16-/Glycidoxydimethicon-Kreuzpolymer, Natriumbutylester vom PVM/MA-Copolymer, Natriumethylester vom PVM/MA-Copolymer, Natrium-Polyacrylat, Natrium-Polygammaglutamat, Soja-Protein-Phthalat, Sterculia urens-Gummi, Terephthalsäure / Isophthalsäure / Natriumisophthalsäuresulfonat / Glykol-Copolymer, Trimethylolpropantriacrylat, Trimethylsiloxysilylcarbamoylpullulan, VA / Crotonate-Copolymer, VA/Crotonate/Methacryloxybenzophenon-1-Copolymer, VA / Crotonate / Vinylneodecanoat-Copolymer, VA / Crotonate /Vinylpropionat-Copolymer, VA/DBM-Copolymer, VA/ Vinylbutylbenzoat / Crotonate-Copolymer, Vinylamin / Vinyl-Alkohol, Vinyl-Caprolactam / VP/ Dimethylaminoethylmethacrylat-Copolymer, VP/Acrylate / Laurylmethacrylat-Copolymer, VP/ Dimethylaminoethylmethacrylat-Copolymer, VP/DMAPA-Acrylate-Copolymer, VP/ Hexadeken-Copolymer, VP/Methacrylamid/Vinylimidazol-Copolymer, VP/VA-Copolymer, VP/Vinyl-Caprolactam / DMAPA-Acrylate-Copolymer, Hefe-Palmitat, ein Silizium-basiertes Polymer oder Harz wie Phenylpropyldimethylsiloxysilikat, Trimethylsiloxysilikat, Cyclopentasiloxan, Trimethylsiloxysilicat, Diisostearoyl, Trimethyllolpropansiloxysilikat, Vinyldimethicon-Kreuzpolymer/Mischungen, Alkylcetearyldimethicon-Kreuzpolymere, und Gemische derselben.

## Revendications

1. Composition émulsifiante comprenant :
au moins environ 40 pour cent en masse d'alcool, par rapport à la masse totale de la composition alcoolique ; et
un tensioactif émulsifiant sélectionné au sein du groupe constitué par les tensioactifs de type polymère de siloxane, les tensioactifs fluorés anioniques, cationiques et zwitterioniques, et leurs combinaisons ; et
un stabilisateur de mousse cationique polymère ou oligomère.

2. Procédé de réduction d'écoulement d'une composition depuis un distributeur de mousse à pompe adapté à la distribution de ladite composition, le procédé comprenant :
le fait de se munir d'une composition émulsifiante comprenant au moins environ 40 pour cent en masse d'alcool, par rapport à la masse totale de la composition alcoolique ; un tensioactif émulsifiant sélectionné au sein du groupe constitué par les tensioactifs de type polymère de siloxane, les tensioactifs fluorés anioniques, cationiques et zwitterioniques, et leurs combinaisons ; et un stabilisateur de mousse polymère ou oligomère ;
le fait de se munir d'un distributeur de mousse à pompe doté d'une tête de distribution ;
le placement de ladite composition émulsifiante dans ledit distributeur de mousse à pompe ; et
la distribution d'un échantillon de ladite composition émulsifiante, une portion dudit échantillon restant dans la tête de distribution pendant au moins 3 minutes, et ladite portion qui reste dans ladite tête de distribution ne coulant pas du distributeur.

3. Procédé de stabilisation d'une mousse formée à partir d'une composition alcoolique émulsifiante, le procédé comprenant :
le fait de se munir d'une composition comprenant un alcool et un tensioactif émulsifiant ; et la combinaison de la composition alcoolique à un stabilisateur de mousse cationique polymère ou oligomère pour former une mousse stabilisée, la stabilité de la mousse stabilisée étant supérieure à la stabilité d'une mousse qui ne contient pas de stabilisateur de mousse, et la stabilité de ladite mousse stabilisée à température ambiante étant d'au moins environ 30 secondes.

4. Composition selon la revendication 1, ou procédé selon la revendication 2 ou 3, où ledit tensioactif émulsifiant comprend un composé tensioactif fluoré pouvant être représenté par la formule
[F₃CF₂C - (CF₂CF₂)ₓ -CH₂CH₂-O-P₂O₃] - [ R¹] ⁺
où [R¹] + inclut DEA, TEA, NH4 ou la bétaïne, et où x est un entier compris entre environ 4 et environ 18 inclus.

5. Composition selon la revendication 1, ou procédé selon la revendication 2 ou 3, où ledit tensioactif émulsifiant comprend un tensioactif de type polymère de siloxane pouvant être représenté par la formule
R² - Si (CH₃)₂ - [O-Si (CH₃)₂]ₐ - [O - Si (CH₃) R³ ]_{b} - O - Si (CH₃)₂ - R²
où R² et R³ incluent indépendamment un groupement méthyle ou un groupement pouvant être représenté par la formule
-(CH₂)₃ - O - (CH₂CH₂O)_{c} - [CH₂CH(CH₃)O]_{d} - (CH₂CH₂O)ₑ H
à la condition que R² et R³ soient tous deux différents de CH₃, où a est un entier compris entre environ 3 et environ 21 inclus, b est un entier compris entre environ 1 et environ 7 inclus, c est un entier compris entre environ 0 et environ 40 inclus, d est un entier compris entre environ 0 et environ 40 inclus et e est un entier compris entre environ 0 et environ 40 inclus, à la condition que a ≥ 3 x b et que c + d + e ≥ 5.

6. Composition selon la revendication 1, ou procédé selon la revendication 2 ou 3, où ledit tensioactif de type polymère de siloxane comprend un tensioactif sélectionné au sein du groupe constitué par les composés suivants : organopolysiloxane-diméthicone-polyols, carbinol-silicones fluides, polyéthers de silicone, alkylméthylsiloxanes, amodiméthicones, éthoxylates de trisiloxane, diméthiconols, tensioactifs de silicone quaternaire, polysilicones, polymères croisés de silicone, cires de silicone, et leurs mélanges.

7. Composition selon la revendication 1, ou procédé selon la revendication 2 ou 3, où ledit tensioactif de type polymère de siloxane comprend les composés suivants : undecylénate de diméthicone PEG-7, diméthicone PEG-10, diméthicone PEG-8, diméthicone PEG-12, perfluorononyléthylcarboxydécal PEG-10, diméthicone PEG-20/PPG-23, éther méthylique de diméthicone PEG-11, diméthicone bis-PEG/PPG-20/20, silicones quaternaires, diméthicone PEG-9, diméthicone PPG-12, diméthicone fluoro-PEG-8, diméthicone PEG 23/PPG 6, diméthicone PEG 20/PPG 23, diméthicone PEG 17, méthicone PEG5/PPG3, diméthicone bis-PEG20, un mélange de copolyol de diméthicone PEG/PPG20/15 et de sulfosuccinate, ou leurs mélanges.

8. Composition selon la revendication 1, ou procédé selon la revendication 2 ou 3, où ledit stabilisateur de mousse comprend un polyquaternium, un copolymère quaternaire de vinylpyrrolidone et de diméthylaminométhacrylate, un copolymère quaternaire modifié hydrophobe de vinylpyrrolidone et de diméthylaminopropylméthacrylamide, ou leurs mélanges.

9. Composition selon la revendication 1, ou procédé selon la revendication 2 ou 3, où ledit stabilisateur de mousse comprend un terpolymère de vinylcaprolactame (VCL), de vinylpyrrolidone (VP) et d'acrylate de dialkylaminoalkyle, ou un copolymère isobutylène/diméthylaminopropylmaléimide/maléimide éthoxylé/acide maléique.

10. Composition antimicrobienne comprenant :
au moins environ 50 pour cent en masse d'alcool, par rapport à la masse totale de la composition alcoolique ;
entre environ 0,002 et environ 4 % en masse d'un tensioactif comprenant un tensioactif fluoré sélectionné au sein du groupe constitué par les composés représentés par la formule
[F₃CF₂C - (CF₂CF₂)ₓ -CH₂CH₂-O-P₂O₃] - [ R¹] ⁺
où [R¹] + inclut DEA, TEA, NH4 ou la bétaïne, et où x est un entier compris entre environ 4 et environ 18 inclus, ou un tensioactif de type polymère de siloxane, sélectionné au sein du groupe constitué par les composés représentés par la formule
R² - Si (CH₃)₂ - [O-Si (CH₃)₂]ₐ - [O - Si (CH₃) R³]_{b} - O - Si (CH₃)₂ - R²
où R² et R³ incluent indépendamment un groupement méthyle ou un groupement pouvant être représenté par la formule
-(CH₂)₃ - O - (CH₂CH₂O)_{c} - [CH₂CH(CH₃)O]_{d} - (CH₂CH₂O)ₑ H
à la condition que R² et R³ soient tous deux différents de CH₃, où a est un entier compris entre environ 3 et environ 21 inclus, b est un entier compris entre environ 1 et environ 7 inclus, c est un entier compris entre environ 0 et environ 40 inclus, d est un entier compris entre environ 0 et environ 40 inclus et e est un entier compris entre environ 0 et environ 40 inclus, à la condition que a ≥ 3 x b et que c + d + e ≥ 5, ou leurs combinaisons ; et
entre environ 0,005 et environ 4 % en masse d'un stabilisateur de mousse cationique polymère ou oligomère.

11. Composition émulsifiante comprenant :
au moins environ 40 pour cent en masse d'alcool, par rapport à la masse totale de la composition alcoolique ; et
un tensioactif émulsifiant sélectionné au sein du groupe constitué par les tensioactifs de type polymère de siloxane, les tensioactifs fluorés anioniques, cationiques et zwitterioniques, et leurs combinaisons ; et un stabilisateur de mousse polymère ou oligomère sélectionné au sein du groupe constitué par les composés suivants : polymères de polyquaternium, terpolymères de vinylcaprolactame (VCL), vinylpyrrolidone (VP) et acrylate de dialkylaminoalkyle, copolymère isobutylène/diméthylaminopropylmaléimide/maléimide éthoxylé/acide maléique, copolymère acrylamide/acrylate d'ammonium, copolymère acrylamides/acrylates de DMAPA/méthoxy-PEG-méthacrylate, copolymère acrylamide/acryloyldiméthyltaurate de sodium/acide acrylique, copolymère chlorure d'acrylamidopropyltrionium/acrylamide, copolymère chlorure d'acrylamidopropyltrimonium/acrylates, copolymère acrylates/méthacrylate d'acétoacétoxyéthyle, copolymère acrylates/acrylamide, copolymère acrylates/méthacrylate d'ammonium, copolymère acrylates/t-butylacrylamide, copolymère acrylates, copolymère acrylates/succinates en C1-2/hydroxyacrylates, copolymère acrylates/méthacrylate d'éthylamine oxyde, copolymère acrylates/acrylate de lauryle /acrylate de stéaryle/méthacrylate d'éthylamine oxyde, copolymère acrylates/octylacrylamide, copolymère acrylates/octylacrylamide/diphénylamodiméthicone, copolymère acrylates/siloxyméthacrylate de polytriméthyle, copolymère acrylates/acrylate de stéaryle/méthacrylate d'éthylamine oxyde, copolymère acrylates/méthacrylate de trifluoropropyle/siloxyméthacrylate de polytriméthyle, copolymère acrylates/VA, copolymère acrylates/VP, copolymère acide adipique/diéthylènetriamine, copolymère acide adipique/diméthylaminohydroxypropyldiéthylènetriamine, copolymère acide adipique/époxypropyldiéthylènetriamine, copolymère acide adipique/acide isophtalique/néopentylglycol/triméthylolpropane, copolymère stéarate d'allyle/VA, copolymère aminoéthylacrylate-phosphate/acrylates, copolymère aminoéthylpropanediol-acrylates/acrylamide, copolymère aminoéthylpropanediol-AMPD-acrylates/diacétoneacrylamide, copolymère VA d'ammonium/acrylates, copolymère amodiméthicone/silsesquioxanne, copolymère AMPD-acrylates/diacétoneacrylamide, copolymère AMP-acrylates/méthacrylate d'allyle, copolymère AMP-acrylates/acrylates d'alkyle en C1-18 /(alkyle en C1-8)-acrylamide, copolymère AMP-acrylates/diacétoneacrylamide, copolymère AMP-acrylates/diméthylaminoéthylméthacrylate, filtrat de fermentation bacille/extrait de son de riz/extrait de soja, copolymère méthacrylate de béhényle/méthacrylate d'éthylamine oxyde, copolymère bis-butyloxyamodiméthicone/PEG-60, copolymère bis-isobutyl-PEG-14/amodiméthicone, copolymère bis-isobutyl-PEG-15/amodiméthicone, copolymère acrylate de butyle/méthacrylate d'éthylhexyle, copolymère acrylate de butyle/acrylate d'hydroxypropyldiméthicone, copolymère ester butylique d'éthylène/MA, copolymère ester butylique de PVM/MA, copolymère calcium/PVM sodium/MA, lactate de chitosane, copolymère amidon de maïs/acrylamide/acrylate de sodium, copolymère diéthylène glycolamine/épichlorohydrine/pipérazine, polymère croisé dediméthicone, copolymère diméthicone/silsesquioxanne, diphénylamodiméthicone, copolymère ester éthylique de PVM/MA, copolymère chlorure d'éthyltrimonium-méthacrylate/hydroxyéthylacrylamide, polymère croisé protéine de blé hydrolysée/PVP, copolymère hydroxypropyldiméthiconylpropyle acrylates, hydroxypropyltrimonium/amidon de maïs hydrolysé, copolymère isobutylène/éthylmaléimide/hydroxyéthylmaléimide, copolymère isobutylène/MA, copolymère isobutylméthacrylate/trifluoroéthylméthacrylate/diméthi cone-acrylate de bis-hydroxypropyle, copolymère d'ester isopropylique de PVM/MA, polymère croisé d'acrylate de lauryle, polymère croisé méthacrylate de lauryle/diméthacrylate de glycol, lauryl-PEG-9-polydiméthylsiloxyéthyl-diméthicone, copolymère acide méthacrylic/sulfonate d'acrylamidométhylpropane sodium, copolymère méthacryloyl-éthyl-bétaïne/acrylates, copolymère méthoxyamodiméthicone/silsesquioxane, méthoxy-PEG-114/polyepsilon-caprolactone, glycérides myristique/palmitique/stéarique/ricinoléique/éicosanedi oïque, copolymère octylacrylamide/acrylates/méthacrylate de butylaminoéthyle, copolymère PEG-800/alcool polyvinylique, copolymère PEG/PPG-25/25 diméthicone/acrylates, copolymère PEG-8/SMDI, polyacrylamide, polyacrylate-6, polyacrylate-8, polyacrylate-9, polyacrylate-15, polyacrylate-16, polyacrylate-17, polyacrylate-18, polyacrylate-19, polymère croisé polybêta-alanine/acide glutarique, polybutylène téréphtalate, polyester-1, polyéthylacrylate, polyéthylène téréphtalate, polyimide-1, polyméthacryloyl-éthyl-bétaïne, polypentaérythrityl-téréphtalate, polyperfluoroperhydrophénanthrène, copolymère polyquaternium-4/hydroxypropylamidon, polyuréthane-1, polyuréthane-6, polyuréthane-10, polyuréthane-18, polyuréthane-19, polyacétate de vinyle, polyvinylbutyral, polyvinylcaprolactame, polyvinylformamide, polyacétate de vinyle imidazolinium, ester butylique de potassium de copolymère PVM/MA, ester éthylique de potassium de copolymère PVM/MA, copolymère PPG-12/SMDI, copolymère PPG-51/SMDI, copolymère PVM/MA, copolymère PVP/VA/acide itaconique, copolymère PVP/VA/propionate de vinyle, gomme rhizobian, acrylate de colophane, gomme laque, polymère croisé silicone quaternium-16/glycidoxydiméthicone, ester butylique de sodium de copolymère PVM/MA, ester éthylique de sodium de copolymère PVM/MA, polyacrylate de sodium, polygamma-glutamate de sodium, phtalate de protéine de soja, gomme karaya, copolymère acide téréphtalique/acide isophtalique/sulfonate de sodium d'acide isophtalique/glycol, triacrylate de triméthylolpropane, triméthylsiloxysilylcarbamoyl-pullulane, copolymère VA/crotonates, copolymère VA/crotonates/méthacryloxybenzophénone-1, copolymère VA/crotonates/néodécanoate de vinyle, copolymère VA/crotonates/propionate de vinyle, copolymère VA/DBM, copolymère VA/benzoate de vinyle et butyle/crotonates, copolymère vinylamine/alcool vinylique, copolymère vinylcaprolactame/VP/méthacrylate de diméthylaminoéthyle, copolymère VP/acrylates/méthacrylate de lauryle, copolymère VP/méthacrylate de diméthylaminoéthyle, copolymère VP/DMAPA acrylates, copolymère VP/hexadécène, copolymère VP/méthacrylamide/vinylimidazole, copolymère VP/VA, copolymère VP/vinylcaprolactame/DMAPA acrylates, palmitate de levure, un polymère ou une résine siliconés tels que phénylpropyldiméthylsiloxysilicate, triméthylsiloxysilicate, cyclopentasiloxane, triméthylsiloxysilicate, diisostéaroyltriméthyllolpropanesiloxysilicate, polymère croisé et mélanges de vinyldiméthicone, polymères croisés d'alkylcétéaryldiméthicone, et leurs mélanges.
